# EUROPEAN PATENT SPECIFICATION

(11) **EP 2 835 074 B1**
(45) Date of publication and mention of the grant of the patent: **14.08.2019**
(21) Application number: 12873724.4
(22) Date of filing: 02.04.2012
(51) Int. Cl.: A45D 24/36, G01B 3/04, A61B 5/107

(54) **HAIR MEASUREMENT TOOL**
HAARMESSINSTRUMENT
OUTIL DE MESURE DES CHEVEUX

(43) Date of publication of application: 11.02.2015
(73) Proprietor: Park Way Co., Ltd., Tokyo 152-0034 (JP)
(72) Inventor: PARK, Young-soo, Tokyo 152-0034 (JP)
(74) Representative: Müller Hoffmann & Partner
(86) International application number: PCT/JP2012/058951
(87) International publication number: WO 2013/150589

(56) References cited:
- WO-A1-03/061428
- DE-U1- 29 713 107
- DE-U1-202011 001 601
- JP-A- S5 383 861
- JP-A- H08 228 823
- JP-A- S59 118 106
- JP-U- H0 176 704
- JP-U- H0 570 412
- JP-U- S4 732 282
- JP-U- S5 998 805
- JP-U- S53 117 383
- JP-Y1- S1 015 063

## Description

### TECHNICAL FIELD

The present invention relates to a hair measurement tool enabling measurement of hair length or hair angle based on an objective index, and also enabling accurate hair cut at a desired length.

### BACKGROUND ART

Conventionally, when cutting hair at a barber shop or a beauty shop, any tools or devices for measuring hair length are not used, and haircut is normally performed by empirical values or eyeball-measurements. Therefore, a desired hair length before haircut and the actual hair length after the haircut are often different.

Meanwhile, at a practical tuition in a barber school or beauty school, or at a barber/beauty practical examination, haircut at a designated length is sometimes ordered. Since the haircut length varies according to the angle of drawing hair from the scalp, it is very important at which angle, relative to the scalp, the hair should be taken and cut. However, since any appropriate tools or devices for measuring the length or direction (angle) of hair do not exist at present, when haircut in the same content is ordered at the practical tuition or examination, the hair length after haircut by each participant in the tuition or examination is likely to be variable, and moreover, from the viewpoint of measuring persons of tuition or examination result, it is difficult to measure the length and angle of haircut objectively.

For reference, Patent Document 1 as described below discloses a front haircut aid for indicating a rough guide when cutting front hair. Moreover, Fig. 3 of Patent Document 2 as described below discloses a haircut aid for enabling haircut at a designated height according to each part of hair. Moreover, Figs. 5 through 15 of Patent Document 3 as described below disclose a haircut aid to be mounted on the head with combination of wire-shaped members. Furthermore, Patent Documents 4 and 5 disclose stationery ruler-type haircut aids.

WO 03/061428 discloses a comb including a linear scale which is used by the hair stylist to determine the length of hair by placing the comb normal to the scalp of a person whose hair is being cut. By doing so, an end part of the comb will contact the scalp. According to a first embodiment of the comb one end part of the comb has a convex curvature and the other end part of the comb is straight. A second embodiment of the comb shows both end parts of the comb having S-shaped curvatures comprising convex portions and concave portions. The specification does not mention the curvatures of the end parts of the embodiments of the comb.

### [Reference Documents of Conventional Art]

### [Patent Documents]

[Patent Document 1] JP2007-029697A
[Patent Document 2] JP1984-098805U
[Patent Document 3] JP2005-312920A
[Patent Document 4] JP1995-040164U
[Patent Document 5] JP3145522Y

### DISCLOSURE OF THE INVENTION

### [Problems to be Solved by the Invention]

The front haircut aid according to Patent Document 1 as described above is the one for cutting front hair, it is impossible to apply to any part other than the front hair. In addition, according to the front haircut aid the front hair is cut by scissors along a front haircut line, and since the positioning relative to the scissors depends on the ability of user who performs the haircut, it is problematic that the accuracy of haircut length depends on the skill of user. Further, the haircut aid according to Patent Document 2 or 3 covers the head wholly and is grandiose, and the measurements require a long time, therefore it is not suitable to perform haircut at various lengths accurately and easily. In addition, those haircut aids according to Patent Documents 1 through 3 have the problem of incapable of hair angle (direction) measurements.

Meanwhile, the haircut aid according to Patent Document 4 or 5 is essentially provided to cope with stationery purposes, and since a concave-convex structure formed in an edge part does not coincide with the accurate distance from an end part of the structural member, it is problematic that the concave-convex structure cannot be diverted for the purpose of accurate haircut by utilizing this haircut aid for positioning relative to the scissors. In particular, the end part of the stationery ruler is a linear side part, while the scalp having the hair is a curved surface. If, with intention of measurement of hair length by the ruler, the linear side part thereof is placed on the scalp, both are in contact with each other at one point, and the attitude (direction) of the ruler becomes instable (shaky) and is difficult to be fixed. Therefore, it is problematic that the accurate length measurement cannot be performed easily.

In the light of the problems as described above, a purpose of the present invention is to provide a hair measurement tool, forming a concave part at a peripheral part of an elongated member to be placed on the scalp for performing measurement, and at least either one of scale lines for length measurement or angled lines for cut direction is provided on a peripheral part of the concave part, so as to secure a two-point contact state when placed on the scalp, whereby hair measurements at various parts can be performed easily and accurately.

In addition, another purpose of the present invention is to provide a hair measurement tool, forming notches for positioning hairdressing scissors at positions corresponding to the scale lines, so that the haircut can be performed at a measured length accurately.

The above objects are solved by the claimed matter according to claim 1.

### [Means for Solving the Problems]

To achieve the object as described above, a hair measurement tool according to claim 1 is disclosed.

According to the present invention, since the concave part is formed at the peripheral part to be placed on the scalp, the hair measurement tool can maintain the stable state while pressing against the scalp. Namely, when the peripheral part of the concave part is placed on the curved surface of the scalp, since the concave part is in a recessing shape and does not contact the scalp, the peripheral part contacts with scalp by two points each of which is on each side of the concave part. Accordingly, the attitude of the hair measurement tool placed on the scalp becomes stable, and hair length measurement and hair direction (angle) measurement at various parts can be performed easily and accurately.

Moreover, according to the present invention, since the concave part is formed at one end part to be placed on the scalp, the stable pressing state of the hair measurement tool against the scalp can be maintained by securing the two-point contact state. Accordingly, the attitude of the hair measurement tool placed on the scalp becomes stable, and hair length measurement at various parts can be performed easily and accurately.

In addition, according to the present invention, since the one end part is formed to be inclined in the length direction, when the hair measurement tool is placed on (pressed against) the scalp, the hair measurement tool becomes in an attitude standing in the normal line direction corresponding to the angle of the inclined one end part relative to each part of the curved surface of the scalp, whereby usages matching up with the sense of direction of user can be provided.

More in addition, according to the present invention, since the angled lines are indicated at the one end part of the concave part, with the hair measurement tool capable of maintaining the stable attitude of pressing against the scalp, the hair direction measurement can also be performed accurately and easily.

Furthermore, according to the present invention, since a comb part is provided at the one end part, it becomes easier to place the one end part on the scalp by plowing through the hair with the comb part. Accordingly, in the case that the one end part has difficulties in reaching the scalp interrupted by the hair, such as thick hair or long hair, the one end part can be placed on (pressed against) the scalp securely by plowing through the hair with the comb part.

In addition, according to the present invention, since an auxiliary concave part is formed at the peripheral part along the length direction and auxiliary angled lines are also provided at that peripheral part, the haircut direction can be measured by placing the peripheral part of the auxiliary concave part on the scalp. Namely, where the peripheral part of the concave part is placed on the scalp, the hair measurement tool becomes in a lateral state, not in a standing state against the scalp, and since the length direction can be used widely for the direction measurement, a wide range of angle measurement can be performed easily.

Furthermore, according to the present invention, since the concave part is in a curve and the scale lines are curved lines corresponding to the curve, the hair length measurement matching up with the curved surface of the scalp can be performed. Namely, when seeing a hair bundle (hair panel) of a predetermined volume, the hair tip thereof is generally cut in a curved line corresponding to the curved surface of the scalp, and therefore, with the curved lines of the scale lines, natural hairdressing at a desired accurate length can be performed.

In addition, according to the present invention, notches are formed corresponding to the scale lines so as to catch a crest of hairdressing scissors, and therefore, in the state that the one end part is pressed against the scalp as described above, with the positioning of the crest of the hairdressing scissors at the notch corresponding to a desired length, the haircut at the desired length can be performed.

More in addition, according to the present invention, since the notches are formed in whole circumferential surfaces of the elongated member, the positioning of the crest of the hairdressing scissors can be performed in any of the whole circumferential directions of the hair measurement tool, and the haircut at the accurate length can be performed from a preferred direction of user.

And furthermore, according to the present invention, when the length of hair (hair bundle) is measured with the scale lines, it is expected that the hair bundle as the measurement object is often set (placed) on the hair measurement tool, and with prevention of the hair bundle, etc., as the measurement object from dropping out of the hair measurement tool, the member constituting the hair measurement tool is formed by a material capable of elastic deformation to be warped in the width direction, so that the hair measurement tool is deformed by grip strength of the user in a warped state surrounding the hair bundle as the measurement object in the width direction, the hair bundle will not drop out easily, and the length measurement can be performed smoothly.

Furthermore, according to the present invention, the member constituting the hair measurement tool is a long-plate-shaped member folded over to be capable of opening and closing, and therefore, in the open state, an end side of the one end part becomes in a V-shape, and when the one end part is placed on the scalp, three points contact the scalp, and this three-point contact can provide more stable measurement. In addition, by arranging the hair measurement tool so as to accommodate the hair bundle as the measurement object in the hair measurement tool in the open state, the hair bundle will not drop out easily, and the length measurement can be performed smoothly.

### [Effects of the Invention]

According to the present invention, since the concave part is formed at the peripheral part to be placed on the scalp, the stable pressing state of the hair measurement tool against the scalp can be maintained, and hair length measurement or hair direction measurement at various parts can be performed easily and accurately.

Furthermore, according to the present invention, since the concave part is formed at the one end part to be placed on the scalp, the stable pressing state against the scalp can be maintained by securing the two-point contact state, whereby the stable state of the hair measurement tool pressed against the scalp can be maintained, and hair length measurement at various parts can be performed easily and accurately.

According to the present invention, since the one end part is formed to be inclined in the length direction, when the hair measurement tool is placed on the scalp, the hair measurement tool can stand towards prescribed direction corresponding to the inclined angle of the one end part relative to each part of the curved surface of the scalp, whereby the usage matching up with the sense of direction of user can be provided.

According to the present invention, since the angled lines are indicated at the one end part of the concave part, the hair direction measurement can also be performed accurately and easily.

According to the present invention, since the comb part is provided at the one end part, even in the case that the one end part has difficulties in reaching the scalp interrupted by the hair, the one end part can be placed on (pressed against) the scalp securely by plowing through the hair with the comb part.

According to the present invention, since the auxiliary concave part is formed at the peripheral part along the length direction and the auxiliary angled lines are also provided at that peripheral part, a wide range of angle measurement can be performed easily.

According to the present invention, since the concave part is in a curve and the scale lines are curved lines corresponding to the curve, the hair length measurement can be performed corresponding to the curved-surface-shape of the scalp.

According to the present invention, since the notches are formed corresponding to the scale lines so as to catch the crest of the hairdressing scissors, the haircut at the desired length can be performed.

According to the present invention, since the notches are formed in the whole circumferential surfaces of the elongated member, the haircut at the accurate length can be performed from the preferred direction of the user.

According to the present invention, since the member constituting the hair measurement tool is formed by the material capable of elastic deformation to be warped in the width direction, when the hair measurement tool is deformed by grip strength of the user in the warped state surrounding the hair bundle as the measurement object in the width direction, the hair bundle will not drop out easily, and the length measurement can be performed smoothly.

According to the present invention, since the member constituting the hair measurement tool is the long-plate-shaped member folded over to be capable of opening and closing, in the open state, more stable measurement can be performed with the three-point contact with the scalp, and the drop out of the hair bundle during measurement can be prevented securely, whereby the length measurement can be performed smoothly.

### BRIEF DESCRIPTION OF THE DRAWINGS

Fig. 1 is a perspective view schematically showing a hair measurement tool according to an embodiment of the present invention.
Fig. 2(a) is a plan view showing the vicinity of one end part, Fig. 2(b) is a side view showing the vicinity of the one end part, and Fig. 2(c) is a plan view showing the vicinity of the other end part.
Fig. 3(a) is an enlarged view of a notch, Fig. 3(b) is a schematic view of common hairdressing scissors, and Fig. 3(c) is a schematic view showing a state that a crest at the back of a cutting blade of the hairdressing scissors is caught by the notch.
Fig. 4(a) is a schematic view showing a state that the hair measurement tool is pressed against the scalp, as seen from the side of a head, Fig. 4(b) is a schematic view showing a state that the hair measurement tool is pressed against the scalp, as seen from the front of the head.
Fig. 5 is a schematic view showing a state that the hair measurement tool is pressed against the scalp, as seen from the top of the head.
Figs. 6(a) and (b) are enlarged schematic views of the vicinity of the one end part, showing the states that the hair measurement tool is pressed against the scalp.
Fig. 7(a) is a schematic view showing a state that the length of hair bundle is measured by the hair measurement tool, and Fig. 7(b) is a schematic view showing a state that the positioning of the hairdressing scissors is performed by using the hair measurement tool and the hair bundle is cut.
Fig. 8(a) is a plan view showing a substantial part of the hair measurement tool according to a first modification type, and Fig. 8(b) is a side view showing a substantial part of the hair measurement tool according to a second modification type.
Fig. 9(a) is a plan view showing the vicinity of the one end part of the hair measurement tool according to a third modification type, Fig. 9(b) is a plan view showing the vicinity of the other end part of the hair measurement tool according to the third modification, and Fig. 9(c) is a plan view showing the vicinity of the one end part of the hair measurement tool according to a fourth modification type.
Fig. 10 shows a hair measurement tool according to a fifth modification type, where Fig. 10(a) is a plan view showing the vicinity of the one end part, and Fig. 10(b) is a schematic perspective view showing a state of pressing against the scalp by plowing through the hair.
Fig. 11(a) is a plan view showing the vicinity of the one end part of the hair measurement tool according to a sixth modification type, Fig. 11(b) is a plan view showing the vicinity of the one end part of the hair measurement tool according to a seventh modification type, and Fig. 11(c) is a plan view showing the vicinity of the other end part of the hair measurement tool according to the seventh modification type.
Fig. 12(a) is a schematic view showing a state that the hair measurement tools according to the seventh modification type are pressed against the scalp, as seen from the side of the head, and Fig. 12(b) is a schematic view showing a state that the hair measurement tools according to the seventh modification type are pressed against the scalp, as seen from the front of the head.
Fig. 13 shows a hair measurement tool according to an eighth modification type, where Fig. 13(a) is a schematic perspective view showing a warped state in the width direction, Fig. 13(b) are schematic views showing the transformation from a flat state to the warped state, as seen from an end surface of an end part, and Fig. 13(c) is a schematic view showing a state that the hair bundle is set, as seen from the end surface of the end part.
Fig. 14 shows a hair measurement tool according to a ninth modification type, where Fig. 14(a) is a schematic perspective view showing an open state, Fig. 14(b) is a side view showing a closed state, and Fig. 14(c) is cross-sectional views showing the transformation from the closed state to the open state, taken along the line B-B of Fig. 14(b).
Fig. 15 is a schematic perspective view showing a state that the hair measurement tool according to the ninth modification type is pressed against the scalp.
Fig. 16 shows a hair measurement tool according to a tenth modification type, where Fig. 16(a) is a plan view showing the vicinity of one end part, and Fig. 16(b) is a plan view showing the vicinity of the other end part.
Fig. 17 shows a hair measurement tool according to an eleventh modification type, as seen from one surface, where Fig. 17(a) is a plan view showing the vicinity of one end part, Fig. 17(b) is a plan view showing the vicinity of the other end part, and Fig. 17(c) is a schematic plan view showing a state of inclination of the one end part.
Fig. 18 shows the hair measurement tool according to the eleventh modification type, as seen from the other surface, where Fig. 18(a) is a plan view showing the vicinity of the one end part, Fig. 18(b) is a plan view showing the vicinity of the other end part, and Fig. 18(c) is a schematic plan view showing a state of inclination of the other end part.
Fig. 19 shows the hair measurement tool according to the eleventh modification type, where Fig. 19(a) is a plan view showing angled lines on the one surface between the both end parts, and Fig. 19(b) is a plan view showing angled lines on the other surface between the both end parts.
Fig. 20(a) is a schematic view showing a state that the hair measurement tools according to the eleventh modification type are pressed against the scalp, as seen from the side of the head, and Fig. 20(b) is a schematic view showing a state that the hair measurement tools according to the eleventh modification type are pressed against the scalp, as seen from the front of the head.
Fig. 21(a) is a schematic view showing a state that the one end parts of the hair measurement tools according to the eleventh modification type are pressed against the scalp, as seen from the top of the head, and Fig. 21(b) is a schematic view showing a state that the other end parts of the hair measurement tools according to the eleventh modification type are pressed against the scalp, as seen from the top of the head.
Fig. 22 shows a hair measurement tool according to a twelfth modification type, where Fig. 22(a) is a plan view of one surface, and Fig. 22(b) is a plan view of the other surface.
Fig. 23 shows states that auxiliary concave parts of the hair measurement tools according to the twelfth modification type are placed on the scalp, where Fig. 23(a) is a schematic view as seen from the side of the head, and Fig. 23(b) is a schematic view as seen from the front of the head.
Fig. 24 shows the hair measurement tool according to the twelfth modification type, where Fig. 24(a) is a schematic view showing a state that the auxiliary concave parts of the hair measurement tool according to the twelfth modification type are placed on the scalp, as seen from the top of the head, and Fig. 24(b) is an enlarged schematic view showing the vicinity of the auxiliary concave part placed on the scalp.

### MODES FOR CARRYING OUT THE INVENTION

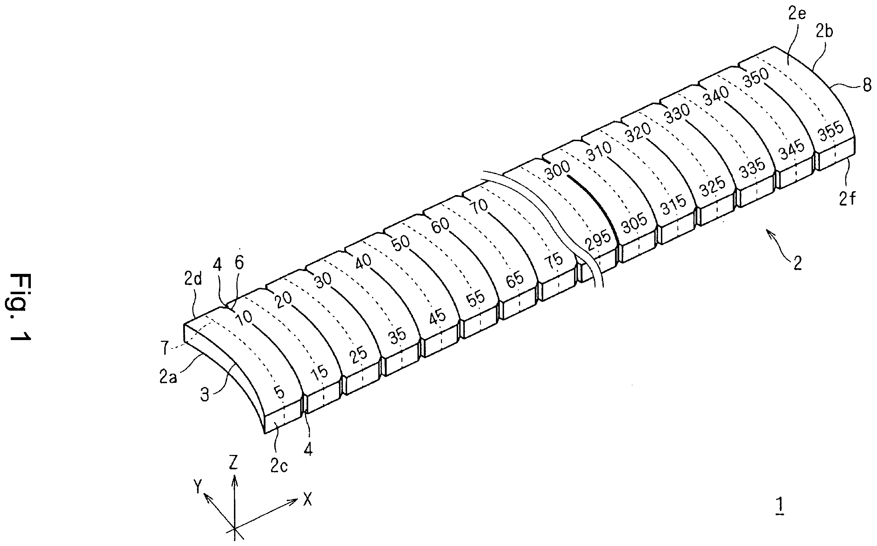

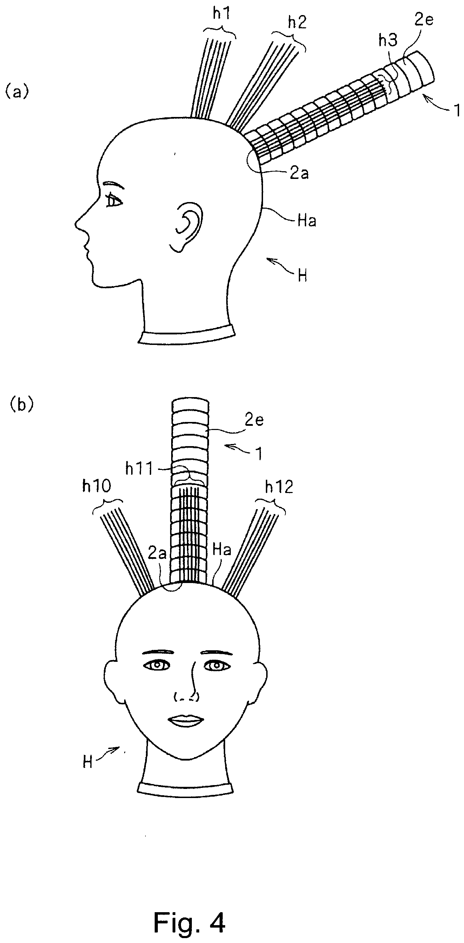

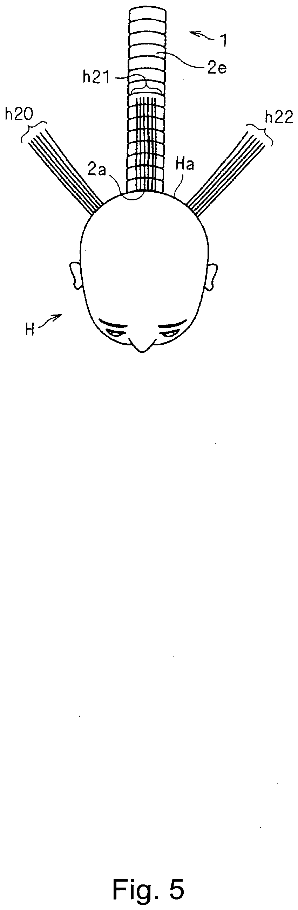

Fig. 1 is a perspective view schematically showing an overview structure of a hair measurement tool 1 according to an embodiment of the present invention. As illustrated in Figs. 4 and 5, the hair measurement tool 1 is used by placing thereof on the scalp of head having hair (crine), which enables accurate measurement of hair or hair bundle of the head (hair panel), measurement of direction in which the hair is to be stretched during the measurement of length (direction in which the hair is taken), and accurate haircut at a desired length.

As illustrated in Fig. 1, the hair measurement tool 1 according to the present embodiment is comprised of an elongated member 2 formed by a flat, long-plate shape made of synthetic resin, and the overview appearance is similar to a common stationery ruler. As different points therefrom, at one end part 2a (corresponding to one section of a peripheral part) of the elongated member 2 constituting the hair measurement tool 1, a curved concave (concave part 3) is formed so that the center in the width directions thereof is the deepest, scale lines 6 are indicated on one surface 2e with intervals at a predetermined length unit from the one end part 2a in conformity with the curve of the concave part 3, and notches 4 are formed in both side surfaces 2c and 2d corresponding to each of the scale lines 6. Note that the X-axis direction as illustrated in Fig. 1 is a length direction of the hair measurement tool 1, the Y-axis direction is a width direction perpendicular to the length direction of the hair measurement tool 1, and the Z-axis direction is a height direction of the hair measurement tool 1 (the direction perpendicular to both the length direction and the width direction, i.e., the thickness direction of the hair measurement tool 1). The same applies to other drawings. Below, the hair measurement tool 1 will be explained in detail.

The hair measurement tool 1 of the present embodiment has the longitudinal length (in the X-axis direction) of about 360 mm, and the lateral width (in the Y-axis direction) of 50 mm (in Fig. 1, the hair measurement tool 1 is illustrated by cutting at the intermediate part in the length direction). Although the size of the hair measurement tool 1 is not limited to the above values (length 360 mm, width 50 mm), when taking account of measurement of hair bundle set on the hair measurement tool 1, it is desirable to secure the width larger than approximately 20 mm. When taking account of user-friendliness (easiness of handling) of the hair measurement tool 1 with also securing easiness of setting of hair bundle, it is desirable to set the ratio of the length and the width, when the ratio of the width is "1," to be within the range of length: width = "about 10 - about 5": "1."

Moreover, the material of the hair measurement tool 1 is not limited to synthetic resin, and for example, any natural material such as wood or bamboo can also be applied. Furthermore, although the elongated member 2 of which thickness is in the range of about 0.5 mm - about 5 mm can be adopted as the hair measurement tool 1, when taking account of easiness of holding and required stiffness, etc., the desirable thickness is about 1 - 2 mm.

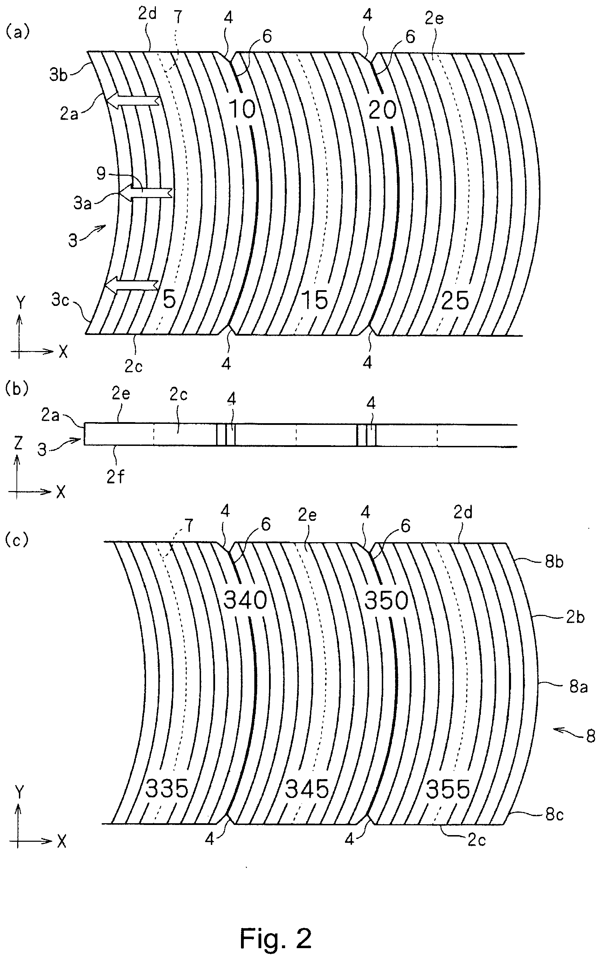

As described above with the drawing of Fig. 2(a), the concave part 3 is formed in an end surface of the one end part 2a. The concave part 3 has a radius of 100 to 120 mm, which is a slightly bigger curvature than the biggest curvature part of common human head.

Furthermore, the hair measurement tool 1 indicates the scale lines 6 on the one surface 2e (applied to the one surface 2e), in solid lines (thickness about 0.6 mm) from the one end part 2a, with intervals at 10 mm. These scale lines 6 are curved lines having the same curvature as that of the concave part 3. Furthermore, the hair measurement tool 1 indicates intermediate scale lines 7 in dashed lines, at the intermediate position between the one end part 2a and the first scale line 6, and another intermediate position between the second scale line 6 and the subsequent scale line 6, and so on, so that the intermediate scale lines are indicated between all the adjacent scale lines 6. These intermediate scale lines 6 are also curved lines likewise the scale lines 6. In addition, at every position in increments of 1 mm from the one end part 2a (excluding the positions of the scale lines 6 and the intermediate scale lines 7), 1-mm scale lines, which are thinner lines than each of the scale lines 6, are indicated (the 1-mm scale lines are also curved lines likewise the scale lines 6 and the intermediate scale lines 7). With such a curve of each of the scale lines 6, 7, etc., having the same curvature as that of the concave part 3, the measurement can be performed in a state that the hair bundle has the naturally curved hair tips. Note that, with reference to the drawings other than Figs. 2(a) and (c), for the purpose of avoiding complicated illustration content, the indication of 1-mm scale lines are omitted (in Figs. 4, 5, etc., the intermediate scale lines 7 are also omitted). Furthermore, the 1-mm scale lines vary the line thickness within the range of 10 mm unit. The 1-mm scale line closer to the one end part 2a is the thinnest, and as going away from the one end part 2a, each of the 1-mm scale lines becomes thicker. For example, the thickness of the 1-mm scale line closest to the one end part 2a is 0.20 mm, and the second 1-mm scale line is 0.22 mm, the third second 1-mm scale line is 0.24 mm, the fourth 1-mm scale line is 0.26 mm, the sixth 1-mm scale line is 0.28 mm, the seventh 1-mm scale line is 0.30 mm, the eighth 1-mm scale line is 0.32 mm, and the ninth 1-mm scale line is 0.34 mm (the fifth scale line is the dashed line, as described above). With this variation of line thickness of each of the 1-mm scale lines, each 1-mm scale line can be read easily. Note that, to the contrary, it is also possible that the 1-mm scale line closest to the one end part 2a is the thickest, and that the thickness becomes smaller as going away from the one end part 2a.

The hair measurement tool 1 indicates values on the one surface 2e, per scale line 6 as described above, showing the length from the one end part 2a (for example, the first scale line 6 from the one end part 2a indicates "10" as the value showing the length of 10 mm, and the second scale line 6 from the one end part 2a indicates "20" as the value showing the length of 20 mm). In addition, the hair measurement tool 1 also indicates values on the one surface 2e, per intermediate scale line 7 as described above, showing the length from the one end part 2a (for example, the first intermediate scale line 7 from the one end part 2a indicates "5" as the value showing the length of 5 mm, and the second intermediate scale line 7 from the one end part 2a indicates "15" as the value showing the length of 15 mm). Moreover, the hair measurement tool 1 indicates in total three white arrows 9, respectively pointing the side of the one end part 2a, on the side of the one end part 2a of the one surface 2e.

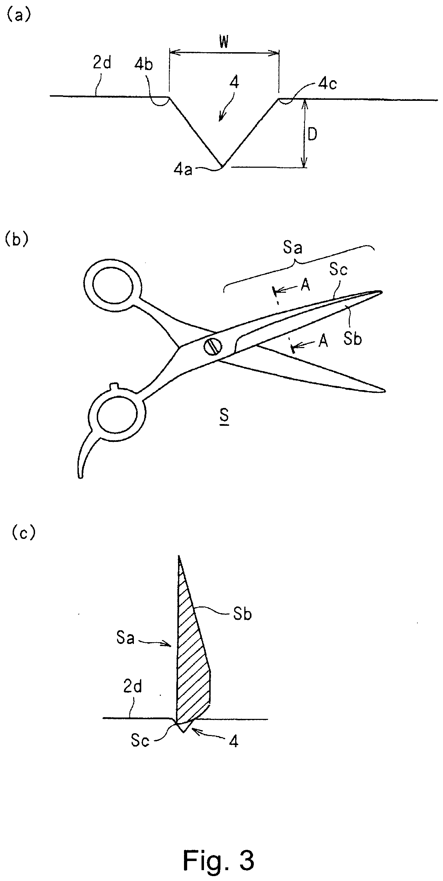

Furthermore, the hair measurement tool 1 has the V-shaped notches 4 formed in both the side surfaces 2c, 2d. Each of the notches 4 is formed exactly at the position corresponding to the scale line 6. Fig. 3(a) is an enlarged view of the notch 4 formed in the one side surface 2d, and the notch 4 according to the present embodiment has about 2 mm of notch width W (opening width) between both end parts 4b, 4c of a notch entrance, and also has about 1.5 mm of notch depth D from the side surface 2d to a bottom peak 4a. With this structure, the crest, that is the back of a cutting blade of the hairdressing scissors, can be caught (hooked) by the notch 4.

Fig. 3(b) illustrates common hairdressing scissors S. The hairdressing scissors S are composed of a member serving as a moving blade, and a member serving as a stationary blade (that is the member in which a finger hook is projecting from a finger ring), overlaid and rotatably connected by a screw, and each of the members serving as the moving blade and the stationary blade, respectively, has the cutting blade on the tip side from a connected position.

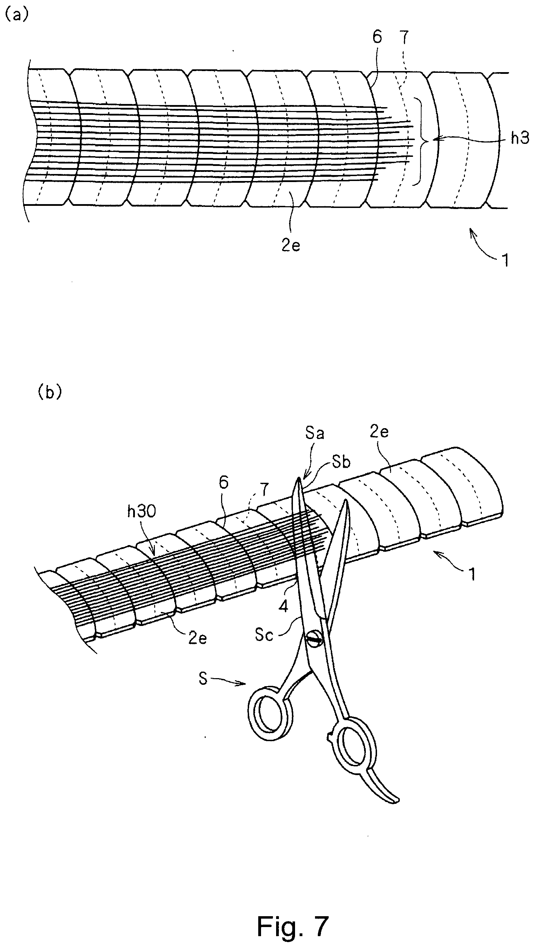

Namely, the member serving as the stationary blade has a cutting blade Sa on the tip side from the connected position, and the cutting blade Sa has a blade tip Sb on the side facing the moving blade, and also has a crest Sc on the opposite side, that is the back side of the cutting blade Sa. As illustrated in Fig. 3(c), the cross section of the blade tip Sb is in a tapered wedge shape, and the thickness of the crest Sc is equivalent to that of the member serving as the stationary blade. Moreover, the cutting blade Sa of Fig. 3(c) is the cross section of Fig. 3(b) as seen by the line A-A, and the cross section is a blade reverse surface having a slight clearance on the side slidably crossing to the moving blade. The crest Sc of this cutting blade Sa has a corner (edge), and when the crest Sc places on the notch 4 of the hair measurement tool 1 as described above, the corner (edge) of the crest Sc is exactly accommodated and caught in the inside of the notch 4, whereby the positioning of the cutting blade Sa of the hairdressing scissors S can be performed (see Fig. 3(c), Fig. 7(b)).

Furthermore, Fig. 2(b) is a view of the hair measurement tool 1 as seen from the one side surface 2c. The plurality of cutout-shaped notches 4 exists in the side surface 2c, and at the same time, the scale lines 6 meet with the bottom peaks 4a of the notches 4, so that the position per 10 mm can be confirmed also from the side surface 2c. Furthermore, the intermediate scale lines 7 in dashed lines are also indicated by extending around the side surface 2c. Moreover, the other side surface 2d of the hair measurement tool 1 has substantially the same structure as that of the one side surface 2c as illustrated in Fig. 2(b).

Fig. 2(c) is an enlarged plan view showing the vicinity of another end part 2b of the hair measurement tool 1. The end surface of the other end part 2b is a projecting concave part 8, in a shape that the concave part 3 of the one end part 2a directly shifts in the X-axis direction, in which a center part 8a in the width direction is the outermost extruding part, while end parts 8b, 8c in the width direction are positioned setting back from the center part 8a. Moreover, the one surface 2e of the other end part 2b has substantially the same structure as that described in Fig. 2(a), namely, the scale lines 6, the intermediate scale lines 7 and the 1-mm scale lines are indicated on the one surface 2e, and the notches 4 are formed in both the side surfaces 2c, 2d corresponding to the respective positions of the scale lines 6.

Furthermore, for another surface 2f of the hair measurement tool 1, that is the opposite side (reverse side) of the one surface 2e, the structure thereof is substantially the same as that illustrated in Figs. 2(a) and (c). Accordingly, the hair measurement tool 1 indicates the scale lines 6 and the intermediate scale lines 7 on the whole circumferential surfaces of the elongated member 2, and consequently, a user can confirm the scale lines 6 and the intermediate scale lines 7 from any side of the hair measurement tool 1, so that the measurement of hair (hair bundle) can be performed.

Figs. 4(a) and (b), as well as Fig. 5 illustrate using states of the hair measurement tool 1 as described above, and in each of these states, as an explanatory example, a head model (commonly referred to as a "wig") for cut exercise is used as a cut object, but the using states in the case of human head are substantially the same. A head model H is a whole model of human head, into which the hair is implanted (moreover, in Figs. 4(a) and (b) and Fig. 5, for the purpose of clarifying the points of using states, the all hair implanted into the head is omitted, and the hair is illustrated only in the necessary parts).

Fig. 4(a) is a perspective view as seen from the side of the head model H, showing the case of measurement in which the one surface 2e of the hair measurement tool 1 and the side of the head model H are aligned with each other. In this measurement, hair bundles (hair panels) h1, h2, and h3 as the measurement objects, of which respective width is about 10 - 50 mm, are picked from the head, and these hair bundles h1, h2 and h3 are measured by the hair measurement tool 1. For example, as illustrated in Fig. 4(a), when the hair bundle h3, positioned in the upper part of the back of the head, is measured, the hair bundle h3 picked from the head is raised from a scalp Ha, and the hair bundle h3 is applied to the one surface 2e of the hair measurement tool 1. At that time, the one end part 2a of the hair measurement tool 1 is placed on (pressed against) the scalp Ha, that is a root position of the hair bundle h3 of the head model H.

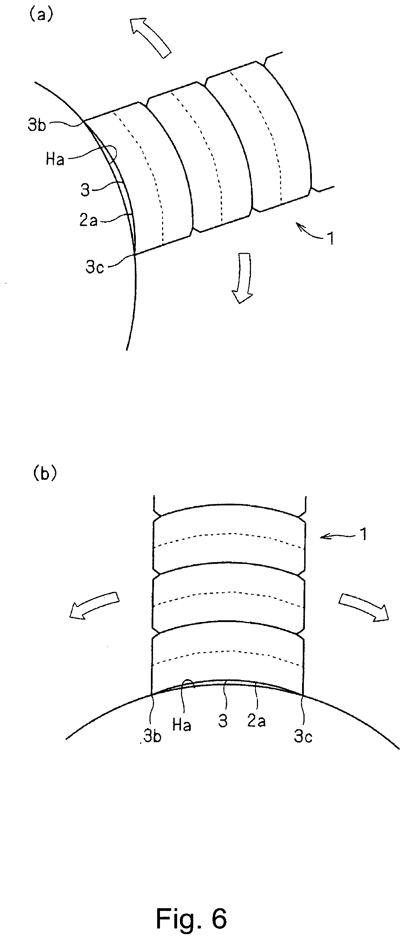

Fig. 6(a) illustrates a state that the one end part 2a of the hair measurement tool 1 is pressed against the scalp Ha in the vicinity of the back of the head (Fig. 4(a) is a view corresponding to the case of measurement of the hair bundle h3). Since the one end part 2a of the hair measurement tool 1 has the concave part 3 formed in the structure as described above, when the one end part 2a is pressed against the scalp Ha, end parts 3b, 3c in the width direction of the concave part 3 become in contact with the scalp Ha. Namely, the curvature of the concave part 3 is slightly bigger than the biggest curvature position of the common human head, and since the curvature of the implanted position of the hair bundle h3 of the head model H is close to the flat surface, a gap from the scalp Ha occurs in the vicinity of the center of the concave part 3, and only the end parts 3b, 3c contact the scalp Ha. Therefore, when the hair measurement tool 1 is pressed against the scalp Ha, both the end parts 3b, 3c become in two-point contact with the scalp Ha, thus the hair measurement tool 1 will not be shaken in the directions of white arrows of Fig. 6(a) (the right and left directions), and the stable attitude can be maintained (the instable state in the white arrow directions will not occur).

Furthermore, Fig. 7(a) illustrates a length measurement state of the hair bundle h3 by the hair measurement tool 1. In this measurement, the length of the hair bundle h3 aligned on the one surface 2e of the hair measurement tool 1, is measured by the scale lines 6 and the intermediate scale lines 7 (or the 1-mm scale lines as described above) indicated on the one surface 2e. Moreover, when a predetermined volume of hair bundle such as the hair bundle h3 is measured, it is desirable to measure by the total average value, not by the unit of single hair. When this measurement is performed, as explained in Fig. 6(a), as long as the one end part 2a of the hair measurement tool 1 is pressed against the scalp Ha, the shaking in the right and left directions will not occur, thus the stable measurement can be performed, and with comparative reference between each of the scale lines 6, 7, etc., and the hair as the measurement object, the length can be measured accurately. In addition, since each of the scale lines 6, 7, etc. are curved lines, the hair tips can be measured along with their curve, thus the trimming of hair tips cut by a natural curved line, in accordance with the curved surface of the scalp Ha, is facilitated.

Moreover, it is also possible to measure the other hair bundles h1, h2 as illustrated in Fig, 4(a), in the same manner as that of the hair bundle h3 described above. Furthermore, the measurement of hair can be performed, not only by the unit of hair bundle (hair panel) as described above, but also, of course, by the unit of one hair, two hairs, and when the measurement by the unit of one hair, two hairs is performed by using the hair measurement tool 1 of the present invention, it is useful for finding the existence of natural hair loss, hair breakage or non-implanted hair part.

Fig. 4(b) is a view as seen from the front of the head model H, showing the case of measurement in which the one surface 2e of the hair measurement tool 1 and the front of the head model H face the same direction. Also in this measurement, hair bundles h10, h11, and h12 as the measurement objects are picked from the head, and these hair bundles h10, h11 and h12 are measured by the hair measurement tool 1, likewise the case of Fig. 4(a).

Fig. 6(b) illustrates a state that the one end part 2a is pressed against the scalp Ha, when the hair measurement tool 1 measures the hair bundle h11 implanted in the vicinity of the top of the head of the head model H of Fig. 4(b). Although the curvature in the vicinity of the top of the head of the head model H is different from the curvature in the vicinity of the back of the head in which the hair bundle h3 is implanted as described in Fig. 6(a), since the curvature of the concave part 3 of the hair measurement tool 1 is slightly bigger than the biggest curvature position of the common human head, also in this case, both the end parts 3b, 3c of the one end part 2a of the hair measurement tool 1 contact the scalp Ha. Accordingly, the hair measurement tool 1 will not be shaken in the directions of white arrows of Fig. 6(b) (the right and left directions), and the stable length measurement can be performed.

Fig. 5 is a view as seen from the top of the head model H, showing the case of measurement in which the one surface 2e of the hair measurement tool 1 and the top of the head model H face the same direction. Also in this measurement, hair bundles h20, h21, and h22 as the measurement objects are picked from the head, and these hair bundles h20, h21 and h22 are measured by the hair measurement tool 1, likewise the case of Fig. 4(a). Also in this case, since the end part 2a of the hair measurement tool 1 being pressed against the scalp Ha becomes in the two-point contact as illustrated in Figs. 6(a) and (b), the stable measurement can be performed. Moreover, Figs. 4(a) and (b) as well as Fig. 5 are examples of length measurement, and substantially the same measurement can be performed at any other position of the head.

Meanwhile, Fig. 7(b) illustrates a cut state of a hair bundle h30 by the hairdressing scissors S, with the hair measurement tool 1. A desired cut length of the hair bundle h30 is measured in the states such as described above in Fig. 4 and Fig. 5, and the crest Sc, that is the opposite side to the blade tip Sb of the cutting blade Sa, is aligned with the notch 4 at the position of the scale line 6 corresponding to the measured cut length. When the crest Sc of the cutting blade Sa is aligned with the notch 4, as explained in Fig. 3(c), the crest Sc of the cutting blade Sa is hooked and caught by the notch 4, and the position of the cutting blade Sa is restricted. Accordingly, the cut position naturally becomes the position indicated by the scale line 6, corresponding to the notch 4 accommodating the crest Sc of the cutting blade Sa, and the accurate cut can be performed at the user's intended length based on the scale line 6.

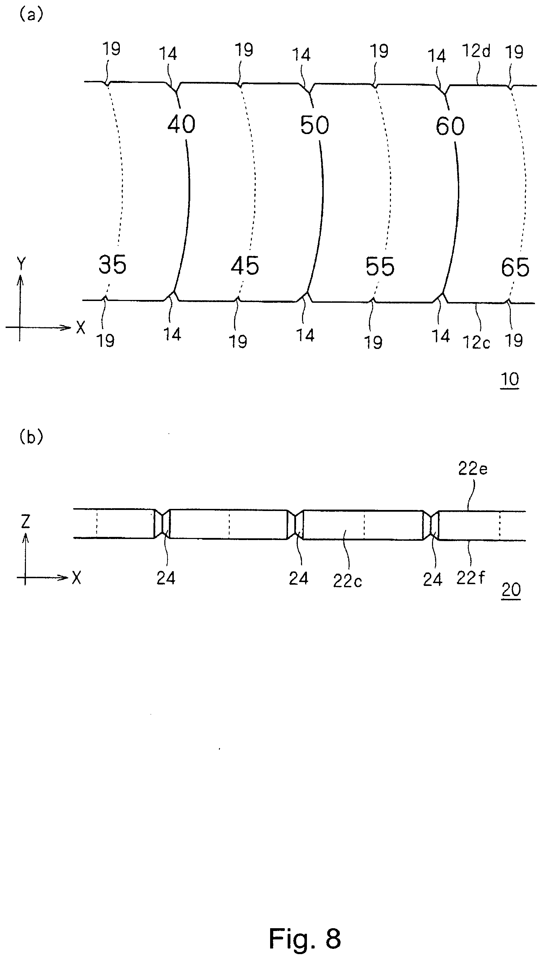

Moreover, the present invention is not limited to the hair measurement tool 1 of the embodiment as described above, and many modifications exist. Fig. 8(a) is a plan view showing essential parts of hair measurement tool 10 of a first modification, characterized in that, in addition to notches 14 formed in side surfaces 12c, 12d corresponding to the scale lines in solid lines, intermediate notches 19 are also formed therein corresponding to the intermediate lines in dashed lines (the other parts are substantially the same as those of the hair measurement tool 1 of Figs. 1, etc.). The intermediate notch 19 is formed in a smaller size than that of the notch 14, for easy distinction from the notch 14 (for example, as compared with the notch 14 having the notch width of about 2 mm and the notch depth of about 1.5 mm, the intermediate notch 19 has the notch width of 1.5 mm and the notch depth of 1 mm). Accordingly, other than with the notches 14, as illustrated in Fig. 7(b), also with the intermediate notches 19, the positioning (position restriction) of the cutting blade Sa of the hairdressing scissors S can be performed, and with the hair measurement tool 10 of the first modification, the accurate cut at more minute unit size can be performed.

Fig. 8(b) is a side view showing essential parts of a hair measurement tool 20 of a second modification, characterized in that each notches 24, provided corresponding to the scale lines in solid lines, are formed in the whole circumferential surfaces of the elongated member constituting the hair measurement tool 20 (the other parts are substantially the same as those of the hair measurement tool 1 of Figs. 1, etc.). Namely, each of the notches 24 in one side surface 22c as illustrated in Fig. 8(b) continues to one surface 22e and another surface 22f (in Fig. 8(b), the positions shown by "V"-shaped recesses in the one surface 22e and the other surface 22f correspond to the notches 24), and furthermore, although not illustrated, the notches 24 also continue to another side surface. With these notches 24 continuously formed in the whole circumferential surfaces, the positioning (position restriction) of the cutting blade of the hairdressing scissors used for haircut can be performed at a desired position by the notch 24 in the whole circumferential direction of the hair measurement tool 20, and when an individual user (beautician, barber) performs haircut from the direction for easily performing haircut, the cut position of the hairdressing scissors can be guided with flexibility.

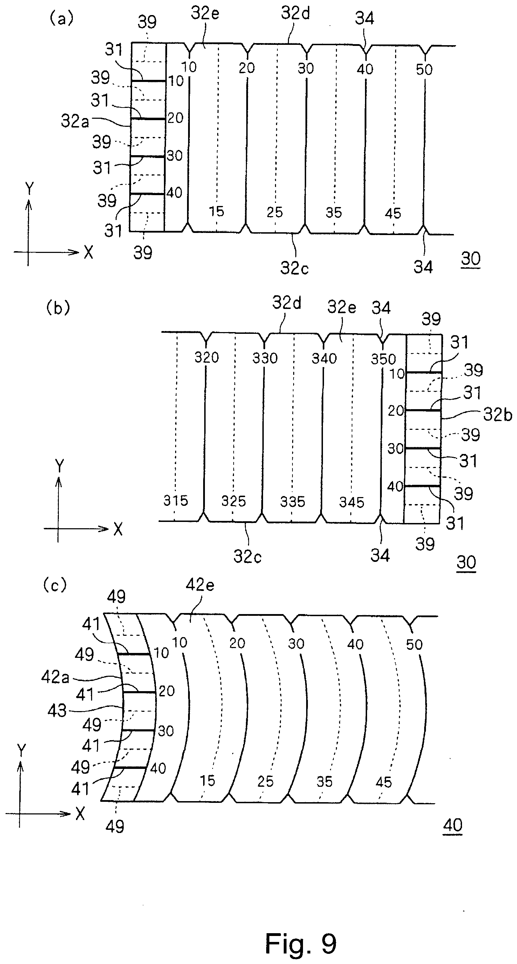

Figs. 9(a) and (b) are plan views showing one end part 32a and another end part 32b of a hair measurement tool 30 of a third modification. Different from the hair measurement tool 1 shown in Figs. 1, etc., the hair measurement tool 30 of the third modification does not have a concave part in the one end part 32a, and has a straight-line shape, with the one end part 32a and the other end part 32b perpendicular to both side surfaces 32c, 32d. Furthermore, each of the scale lines in solid lines and the intermediate scale lines in dashed lines is also a straight line parallel to end surfaces of the one end part 32a and the other end part 32b, with notches 34 formed in the side surfaces 32c, 32d corresponding to the respective scale lines in solid lines.

And furthermore, the hair measurement tool 30 of the third modification indicates, on one surface 32e of the one end part 32a and the other end part 32b, width scale lines 31 in solid lines and width intermediate scale lines 39 in dashed lines, with intervals at a predetermined length unit in the width direction (Y-axis direction).

The width scale lines 31 indicate the length with intervals at 10 mm from the one side surface 32d, and value (10, 20, 30, 40) showing the length from the one side surface 32d is allotted to each of the width scale lines 31. Furthermore, the intermediate width scale lines 39 are indicated at the intermediate position between the one side surface 32d and the first width scale line 31, and also at the intermediate positions between each of the second and subsequent width scale lines 31. With the width scale lines 31 and the intermediate width scale lines 39 provided on the one end part 32a and the other end part 32b, the width of the hair bundle (hair panel) as the measurement object or the cut object can be confirmed by the width scale lines 31 and the intermediate width scale lines 39, whereby the volume of the hair bundle (hair panel) as the measurement object or the cut object can be roughly estimated by the size, that is by the width.

Moreover, the width scale lines 31 and the intermediate width scale lines 39 can be indicated, not only on the one surface 32e, but also on an end surface of the one end part 32a or the other end part 32b, and when the indication is applied to the end surface, it is also possible to confirm the width of the picked hair bundle (hair panel) on the side of the end surface. It is of course possible to indicate the width scale lines 31 and the intermediate width scale lines 39 on the other surface on the reverse side of the one surface 32e. Furthermore, it is also possible to provide the width scale lines 31 and the intermediate width scale lines 39 only on either side of the one end part 32a or the other end part 32b, and when provided in the one end part 32a only, the hair bundle width can be measured on the root side from which the hair is growing, and when provided in the other end part 32b only, the hair bundle width can be measured on the hair tip side.

Fig. 9(c) illustrates one end part 42a of a hair measurement tool 40 of a fourth modification, and the hair measurement tool 40 of the fourth modification is exactly a combination of the hair measurement tool 1 as illustrated in Figs. 1, etc., with the hair measurement tool 30 of the third modification as illustrated in Figs. 9(a) and (b), wherein width scale lines 41 in solid lines and intermediate width scale lines 49 is dashed lines are provided in the one end part 42a, with intervals at a predetermined length unit in the width direction (Y-axis direction) (the other parts are substantially the same as those of the hair measurement tool 1 of Figs. 1, etc.). With the width scale lines 41 and the intermediate width scale lines 49, even at the one end part 42a in a curve, the width of the hair bundle (hair panel) as the measurement object or the cut object can be confirmed.

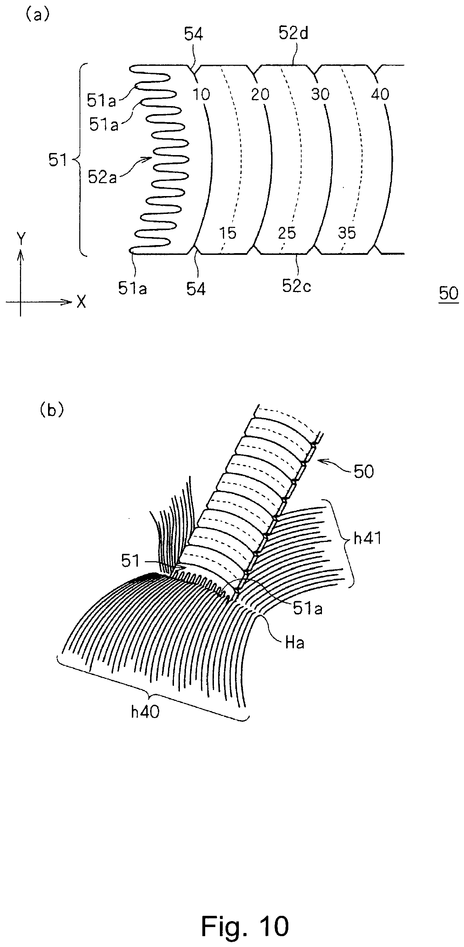

Fig. 10(a) illustrates one end part 52a of a hair measurement tool 50 of a fifth modification, and the hair measurement tool 50 of the fifth modification is exactly in a shape that a comb part is provided in the one end part 2a of the hair measurement tool 1 as illustrated in Figs. 1, etc. Namely, the hair measurement tool 50 is provided with a comb part 51, having a plurality of comb tines 51a, in the one end part 52a, and the other parts are substantially the same as those of the hair measurement tool 1 as illustrated in Figs. 1, etc., with the scale lines in curved solid lines and the intermediate scale lines in curved dashed lines, and also with notches 54 formed in side surfaces 52c, 52d.

The tips of the comb tines 51a of the comb part 51 are aligned along an imaginary curved line, of which curve corresponding to the end surface of the one end part 2a of the concave part 3 of the hair measurement tool 1 as illustrated in Figs. 1, etc. Accordingly, even when the tips of the comb tines 51a are pressed against the scalp Ha of the head as illustrated in Fig. 4 and Fig. 5, likewise the case of the hair measurement tool 1 as illustrated in Figs. 1, etc., the length of hair (hair bundle) can be measured with the two-point contact by the comb tines on both the ends. Since each of the comb tines 51a is pressed against the scalp Ha, when taking account of usage for the human head, it is desirable to form the round-shaped tips to some degree, not to form the steeple-shaped tips. Furthermore, according to the example as illustrated in Fig. 10(a), the tine length of each of the comb tines 51a is about 5 mm, but the tine length is not limited to this example, and it is possible to apply various lengths in the range from approximately 1 mm to approximately 10 mm, and in particular, when the fulfillment of comb function (the function to plow through the hair) is desired without fail, it is desirable to secure the tine length of more than 3 mm.

Fig. 10(b) is a schematic perspective view showing a using state of the hair measurement tool 50. Since the hair measurement tool 50 has the comb part 51 in the one end part 52a, in the case of hair length measurement, when the one end part 52a is pressed against the scalp Ha, it is possible to plow through hair bundles h40, h41 by the comb tines 51a of the comb part 51 provided in the one end part 52a. Furthermore, since each of the individual hairs constituting the plowed-through hair bundles h40, h41 enter the space between each of the comb tines 51a, the tips of the comb tines 51a can contact the scalp Ha. Accordingly, even when the thick hair (hair bundle) of the head is measured, with the plow-through of hair by the comb part 51, the one end part 52a (the tips of the comb tines 51a) of the hair measurement tool 50 of the fifth modification can be pressed against the scalp surely, whereby the hair length can be measured accurately.

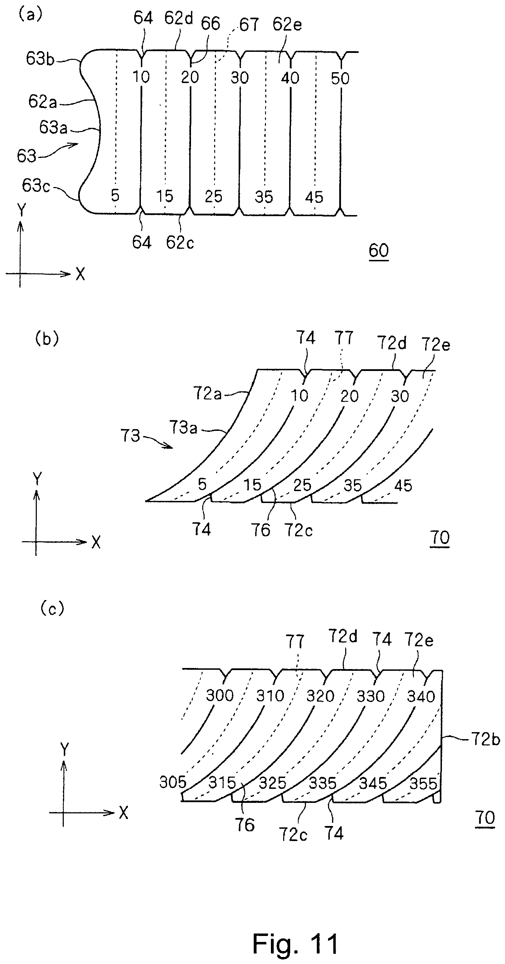

Fig. 11(a) is a plan view showing one end part 62a of a hair measurement tool 60 of a sixth modification. The hair measurement tool 60 of the sixth modification is chiefly a modification of the shape of the concave part 3 of the one end part 2a of the hair measurement tool 1 as illustrated in Figs. 1, etc. Namely, the hair measurement tool 60 is characterized that, both end parts 63b, 63c of a concave part 63 in the width direction (Y-axis direction) are in round shapes (arc shapes with R), not in steeple shapes of the end parts 3b, 3c of the concave part 3 of the hair measurement tool 1 as illustrated in Figs. 1, etc. Furthermore, the curvature (degree of curve) of the concave part 63 is larger than the concave part 3 of Fig. 1, so that a center part 63a of the concave part 63 recesses deeper than that of Fig. 1.

In addition, different from the hair measurement tool 1 as illustrated in Figs. 1, etc., scale lines 66 in solid lines and intermediate scale lines 67 in dashed lines indicated on one surface 62e of the hair measurement tool 60 are straight lines. This is because, since the degree of curve of the concave part 63 is larger than that of the hair measurement tool 1 as illustrated in Figs. 1, etc., if the curved lines as that of the hair measurement tool 1 are applied, the degree of curve of each scale line will become too large, and the length measurement cannot be performed easily. Therefore, for the purpose of avoiding such a situation, the scale lines 66 and the intermediate scale lines 67 are straight lines (the scale lines 66 and the intermediate scale lines 67 are straight lines perpendicular to side surfaces 62c, 62d). Moreover, the other parts of the hair measurement tool 60 is substantially the same as those of the hair measurement tool 1 as illustrated in Figs. 1, etc., with notches 64 formed in the side surfaces 62c, 62d. When the one end part 62a of the hair measurement tool 60 is pressed against the scalp, since both the end parts 63b, 63c of the concave part 63 contacting the scalp are in round shapes, the gentle contact feeling is given to the scalp, and at the same time, since the end parts 63b, 63c become in contact corresponding to the curvature of the scalp, the secure two-point contact can be obtained.

Figs. 11(b) and (c) are plan views showing one end part 72a and another end part 72b of a hair measurement tool 70 of a seventh modification. The one end part 72a of the hair measurement tool 70 of the seventh modification is in an oblique and curve in the length direction (X-axis direction), forming a concave part 73 having a recessed substantial center part 73a. The oblique side of the concave part 73, inclining at the angle of about 45 degrees with respect to the length direction (X-axis direction), has the curvature equivalent to that of the concave part 3 of the hair measurement tool 1 as illustrated in Figs. 1, etc.

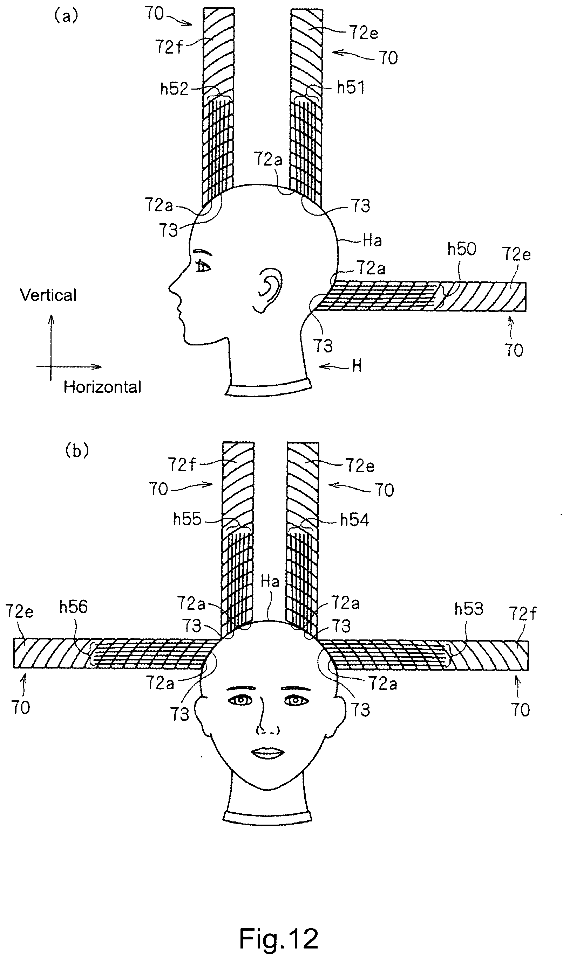

With the concave part 73 of the hair measurement tool 70 of the seventh modification, in a state of being pressed against the head, the attitude of the hair measurement tool 70 is aligned with the absolute vertical direction or the absolute horizontal direction, facilitating the user-friendliness as an index matching up with the sense of direction of the user (beautician or barber). Namely, when the user (beautician or barber) performs haircut or hair setting, he will unconsciously utilize lines indicating the vertical direction or lines indicating the horizontal direction existing around the working space, such as the vertical lines or the horizontal lines of rectangular mirror frames, or the vertical lines or the horizontal lines of cabinet, as the rough index for the substantial vertical direction or the substantial horizontal direction. Therefore, since the position of the hair measurement tool 70 pressed against the scalp can indicate the substantial vertical direction or the substantial horizontal direction, the attitude thereof is aligned with the vertical lines in the vertical direction or the horizontal lines in the horizontal direction existing as the rough index in the working space of the user, matching up with the sense of direction of the user, effective for practical usage (see Figs. 12(a) and (b)).

Furthermore, scale lines 76 in solid lines and intermediate scale lines 77 in dashed lines indicated on one surface 72e of the hair measurement tool 70 are curved lines having the same curvature as that of the concave part 73 (the concave part). Moreover, at the other end part 72b of the hair measurement tool 70, as illustrated in Fig. 11(c), the scale lines 76 and the intermediate scale lines 77 are discontinued, due to their oblique curved lines, on the side of a side surface 72d in the upper part of the drawing. The other parts of the hair measurement tool 70 of the seventh modification are substantially the same as those of the hair measurement tool 1 as illustrated in Figs. 1, etc., and for example, notches 74 are formed in a side surface 72c and the side surface 72d.

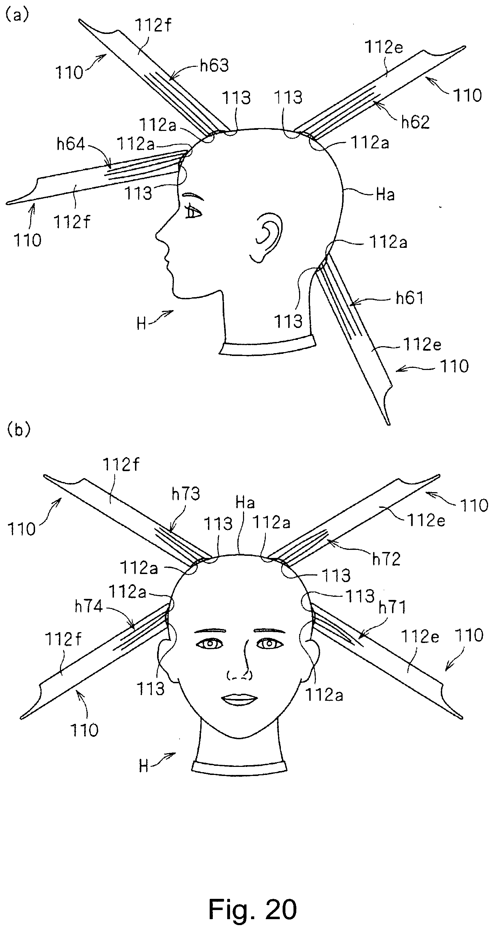

Figs. 12(a) and (b) illustrate using states of the hair measurement tool 70 of the seventh modification for the head model H. Moreover, likewise the cases of Figs. 4(a) and (b) as well as Fig. 5, also in Figs. 12(a) and (b), for the purpose of clarifying the points of using states, the all hair is omitted, and the hair (crine) is illustrated only in the necessary parts (hereinafter, the same applies to Figs. 20, 21, 23 and 24).

Fig. 12(a) is a view as seen from the side of the head model H, showing the case of measurement in which the one surface 72e or another surface 72f of the hair measurement tool 70, and the side of the head model H, are aligned with each other. For example, when the length of a hair bundle h50, implanted in the vicinity of the neck at the back of the head of the head model H, is measured, the one end part 72a of the hair measurement tool 70 is pressed against the scalp in a state that the one surface 72e and the side of the head model H face the same direction. Here, the scalp Ha of the head model H in the vicinity of the neck at the back of the head is an oblique downward curved surface, as illustrated by a left-oriented downward line in Fig. 12(a), and when the one end part 72a of the hair measurement tool 70 is pressed against this downward curved surface of the scalp Ha, because of the shape of the concave part 73 of the one end part 72a, the attitude of the hair measurement tool 70 becomes substantially in the horizontal direction.

In addition, also when the length of a hair bundle h51, implanted in the vicinity of the rear at the top of the head of the head model H, is measured, the one end part 72a of the hair measurement tool 70 is pressed against the scalp Ha in a state that the one surface 72e and the side of the head model H are aligned with each other. The scalp Ha of the head model H in the vicinity of the rear at the top of the head is an oblique upward curved surface, as illustrated by a right-oriented downward line in Fig. 12(a), and when the one end part 72a of the hair measurement tool 70 is pressed against this upward curved surface of the scalp Ha, because of the shape of the concave part 73 of the one end part 72a, the attitude of the hair measurement tool 70 becomes substantially in the vertical direction.

Furthermore, when the length of a hair bundle h52, implanted in the vicinity of the front at the top of the head of the head model H, is measured, the one end part 72a of the hair measurement tool 70 is pressed against the scalp Ha in a state that the other surface 72f and the side of the head model H are aligned with each other. The scalp Ha of the head model H in the vicinity of the front at the top of the head is an oblique upward curved surface, as illustrated by a left-oriented downward line in Fig. 12(a), and when the one end part 72a of the hair measurement tool 70 is pressed against this upward curved surface of the scalp Ha, because of the shape of the concave part 73 of the one end part 72a, the attitude of the hair measurement tool 70 becomes substantially in the vertical direction.

On the other hand, Fig. 12(b) is a view as seen from the front of the head model H, showing the case of measurement in which the one surface 72e or the other surface 72f of the hair measurement tool 70, and the front of the head model H, facing the same direction. For example, when the length of a hair bundle h53, implanted in the vicinity of the left upper part of the head model H, is measured, the one end part 72a of the hair measurement tool 70 is pressed against the scalp Ha in a state that the other surface 72f and the front of the head model H face the same direction. The scalp Ha of the head model H in the vicinity of the upper part at one side of the head is an oblique upward curved surface, as illustrated by a right-oriented downward line in Fig. 12(b), and when the one end part 72a of the hair measurement tool 70 is pressed against this upward curved surface of the scalp Ha, because of the shape of the concave part 73 of the one end part 72a, the attitude of the hair measurement tool 70 becomes substantially in the horizontal direction.

Furthermore, when the length of a hair bundle h54, implanted in the vicinity of the left part at the top of the head model H, is measured, the one end part 72a of the hair measurement tool 70 is pressed against the scalp Ha in a state that the one surface 72e and the front of the head model H face the same direction. The scalp Ha of the head model H in the vicinity of the left part at the top of the head is an oblique upward curved surface, as illustrated by a right-oriented downward line in Fig. 12(b), and when the one end part 72a of the hair measurement tool 70 is pressed against this upward curved surface of the scalp Ha, because of the shape of the concave part 73 of the one end part 72a, the attitude of the hair measurement tool 70 becomes substantially in the vertical direction.

In addition, when the length of a hair bundle h55, implanted in the vicinity of the right part at the top of the head model H, is measured, the one end part 72a of the hair measurement tool 70 is pressed against the scalp Ha in a state that the other surface 72f and the front of the head model H face the same direction. The scalp Ha of the head model H in the vicinity of the right part at the top of the head is an oblique upward curved surface, as illustrated by a left-oriented downward line in Fig. 12(b), and when the one end part 72a of the hair measurement tool 70 is pressed against this upward curved surface of the scalp Ha, because of the shape of the concave part 73 of the one end part 72a, the attitude of the hair measurement tool 70 becomes substantially in the vertical direction.

And furthermore, when the length of a hair bundle h56, implanted in the vicinity of the right upper part of the head model H, is measured, the one end part 72a of the hair measurement tool 70 is pressed against the scalp Ha in a state that the one surface 72e and the front of the head model H face the same direction. The scalp Ha of the head model H in the vicinity of the upper part at the right side of the head is an oblique upward curved surface, as illustrated by a left-oriented downward line in Fig. 12(b), and when the one end part 72a of the hair measurement tool 70 is pressed against this upward curved surface of the scalp Ha, because of the shape of the concave part 73 of the one end part 72a, the attitude of the hair measurement tool 70 becomes substantially in the horizontal direction.

As described above, with the oblique one end part 72a, in a state of being pressed against the head (scalp), since the attitude of the hair measurement tool 70 of the seventh modification can be aligned easily with the substantial vertical direction or the substantial horizontal direction, the usage matching up with the sense of direction of the user (beautician or barber) can be realized.

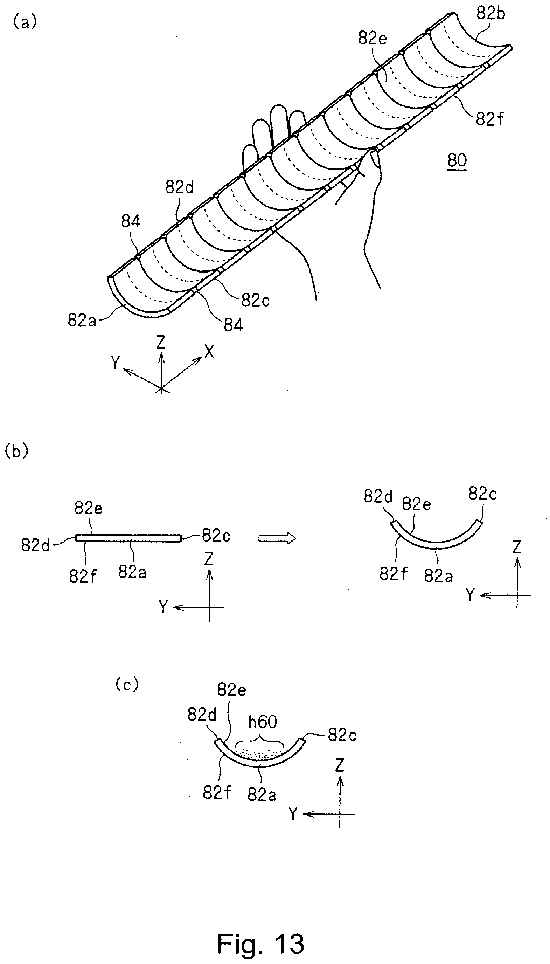

Figs. 13(a) to (c) illustrate a hair measurement tool 80 of an eighth modification. The hair measurement tool 80 is formed by an elongated plate member, made of synthetic resin having flexibility capable of elastic deformation, so as to be warped in the width direction (Y-axis direction). The plate thickness of the hair measurement tool 80 is less than 1 mm, and as illustrated in Fig. 13(a), when the user grasps both side surfaces 82c, 82d and increases the grip strength so as to close the thumb and the index finger to each other, then as illustrated in Fig. 13(b), an end surface of one end part 82a elastically deforms so as to form a warped shape (the hair measurement tool 80 as a whole becomes a gutter shape by elastic deformation). Furthermore, when the grip strength is reduced, it returns to a flat state. Moreover, the other parts of the hair measurement tool 80 of the eighth modification are substantially the same as those of the hair measurement tool 1 as illustrated in Figs. 1, etc., with notches 84 formed in the side surfaces 82c, 82d (a concave part is formed at the one end part 82a placed on the scalp).

With the hair measurement tool 80 having the above characteristics, in the warped state in the width direction as illustrated in Fig. 13(c), when a hair bundle 60 as the length measurement object is placed on one surface 82e, as compared with the flat state, the hair bundle 60 will not drop easily from the one surface 82e, and the stable measurement can be performed. Furthermore, in the warped state as described above, when the one end part 82a of the hair measurement tool 80 is pressed against the scalp of the head, since the end surface of the one end part 82a is curved because of a concave part, both end parts and a center part in the warped state, namely three points in total, become in contact with the scalp, and as compared with the flat state, more stable pressing state can be secured, and also from this viewpoint, the stable measurement can be performed.

Moreover, as a further modification of the eighth modification, the elongated member constituting the hair measurement tool, maintaining the warped state, can be formed by synthetic resin, and with this structure, as illustrated in Fig. 13(a), the hair measurement tool in the warped state, without requiring the grip strength, can be provided. Furthermore, when the hair measurement tool in the warped state, not in the flat state, is formed by synthetic resin, other than the warped state (substantially in "U"-shape), a bent state (deflected to form substantially "V"-shaped bent) can also be formed, and in addition, modifications with various depths of warp (or bent) can be provided.

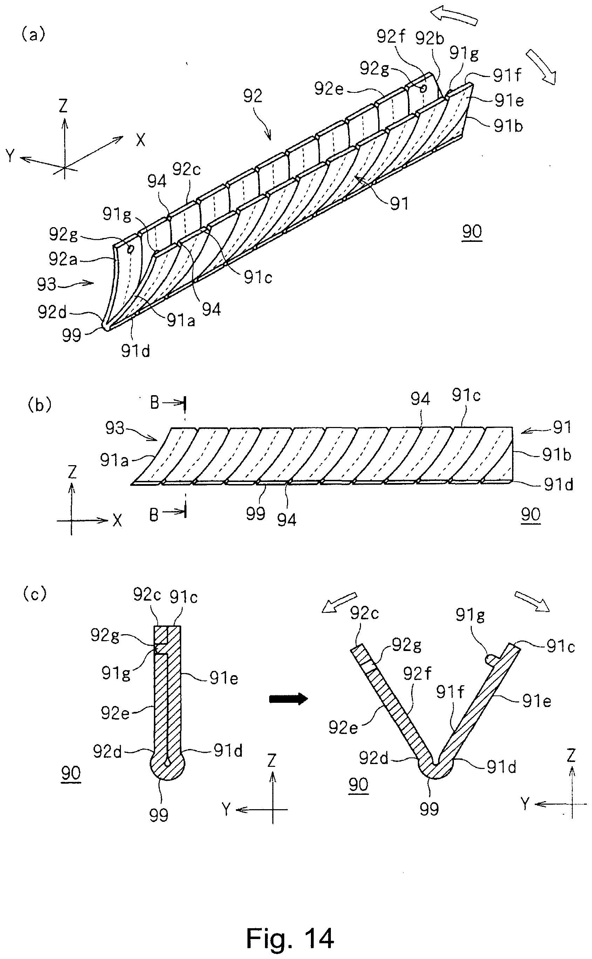

Figs. 14(a) to (c) illustrate a hair measurement tool 90 of a ninth modification. The hair measurement tool 90 is formed by a plate member elongating in the length direction (X-axis direction), in a state of being folded along the length direction. In particular, a first plate part 91 as one part to be folded over, and a second plate part 92 as another part, are connected by one side part 99 elongating in the length direction (a side part on the connecting side of folding over), and the side surfaces 91c, 92d of the respective plate parts 91, 92, namely the side parts on the other side (the side parts on the opening side), can be opened and closed by making the one side part 99 as the pivot. The hair measurement tool 90 with this structure is formed integrally by synthetic resin, and since the one side part 99 connecting both the parts 91, 92 has a hinge-like flexibility, the one side part 99 elastically deforms so as to allow opening and closing of both the parts 91, 92.

The structure of each of the plate parts 91, 92 is essentially equivalent to the hair measurement tool 70 of the seventh modification as illustrated in Figs. 11(b) and (c), with a concave part 93 at one end parts 91a, 92a, also with scale lines in solid lines and intermediate scale lines in dashed lines indicated on one surface 91e, 92e and another surface 91f, 92f, and with notches 94 formed in one side surfaces 91c, 92c.

Furthermore, on other side surfaces 91d, 92d connecting the first plate member 91 and the second plate member 92, the one side part 99 substantially in a slim stick shape is formed, and notches 94 are also formed in this one side part 99 corresponding to each of the positions of the scale lines in solid lines as described above (see Fig. 14(b)).

And furthermore, for the purpose of maintaining the closed state of the first plate part 91 and the second plate part 92, the hair measurement tool 90 has a locking mechanism. The locking mechanism is composed of stick-shaped projections 91g projecting from the other surface 91f in the vicinity of the one end part 91a and in the vicinity of the other end part 91b of the first plate part 91, and locking holes 92g formed in the second plate part 92 so as to face these projections 91g, respectively. The hole diameter of the locking hole 92g and the outer diameter of the projection 91g are determined according to the size relation of so-called "transition fit" or "interference fit" in the field of "fitting" for machine design. Accordingly, when the projections 91g, respectively formed in the vicinity of the one end part 91a and in the vicinity of the other end part 91b, are inserted into the locking holes 92g, respectively formed in the vicinity of the one end part 92a and in the vicinity of the other end part 92b, the projections 91g are fit into (locked by) the locking holes 92g, and the first plate part 91 and the second plate part 92 are folded over and become in an integrated state (see Fig. 14(b) and the drawing on the left of Fig. 14(c)).

Furthermore, from the closed state of the first plate part 91 and the second plate part 92, when each of the side surfaces 91c, 92c is open, the projections 91g come out of the locking holes 92g, and the first plate part 91 and the second plate part 92 are open by making the one side part 99 as the pivot, and the cross section thereof in the width direction becomes in a state of "V" shape (see Fig. 14(c)).

Regardless of the state whether it is open or closed, the hair measurement tool 90 of the ninth modification as described above can be used for hair length measurement, etc., and in the closed state, it is possible to use substantially the same manner as that of the hair measurement tool 70 of the seventh modification, as illustrated in Figs. 11(b) and (c) as well as in Figs. 12(a) and (b).

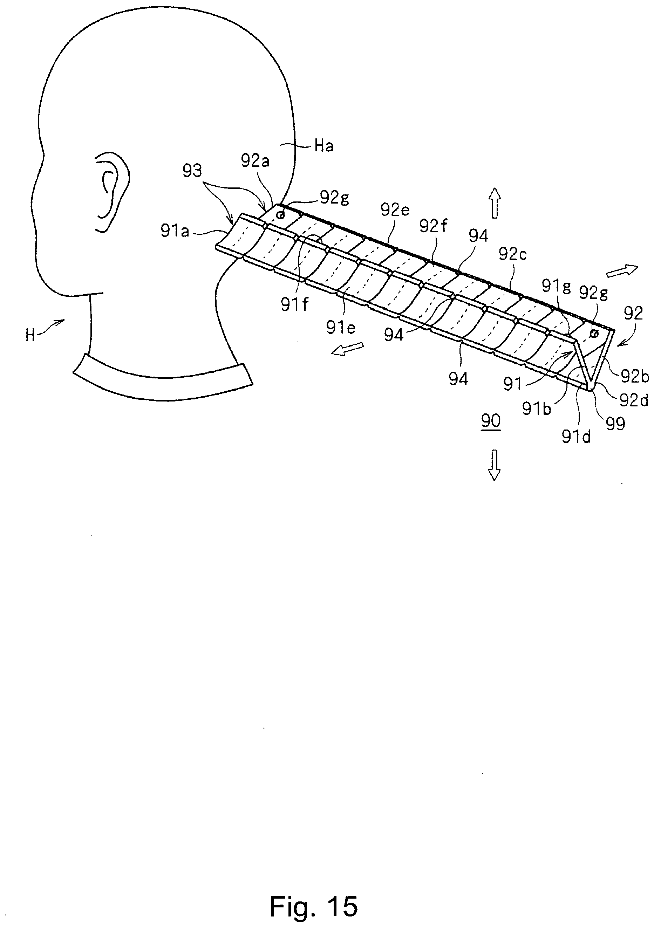

Furthermore, Fig. 15 illustrates a state that the one end parts 91a, 92a of the hair measurement tool 90 in the open state are pressed against the scalp Ha in the vicinity of the neck at the back of the head of the head model H. In this case, since the hair measurement tool 90 is open, at the one end parts 91a, 92a pressed against the scalp Ha, the two end parts of a curved oblique side part 93 on the sides of the side surfaces 91c, 92c, and the end surface of the one side part 99, in total three points, become in contact with the scalp Ha. Therefore, the hair measurement tool 90 in the pressed state will not be shaken in the upward/downward or right/left directions (the directions of white arrows of Fig. 15), thus the stable attitude can be maintained, and more stable length measurement can be performed.

In addition, in the open state of "V"-shape, since the hair or hair bundle as the measurement object can be accommodated in a space between the first plate part 91 and the second plate part 92, the drop-off of the hair or hair bundle as the measurement object can be prevented, and the stable measurement can be performed. Moreover, since the one end parts 91a, 92a of the hair measurement tool 90 of the ninth modification form the curved oblique side part 93, likewise the case of the hair measurement tool 70 of the seventh modification as illustrated in Figs. 12(a) and (b), it is also possible to be pressed against the head in the attitude aligning with the vertical direction or the horizontal direction, whereby the usage matching up with the sense of direction of the user (beautician or barber) can be realized.

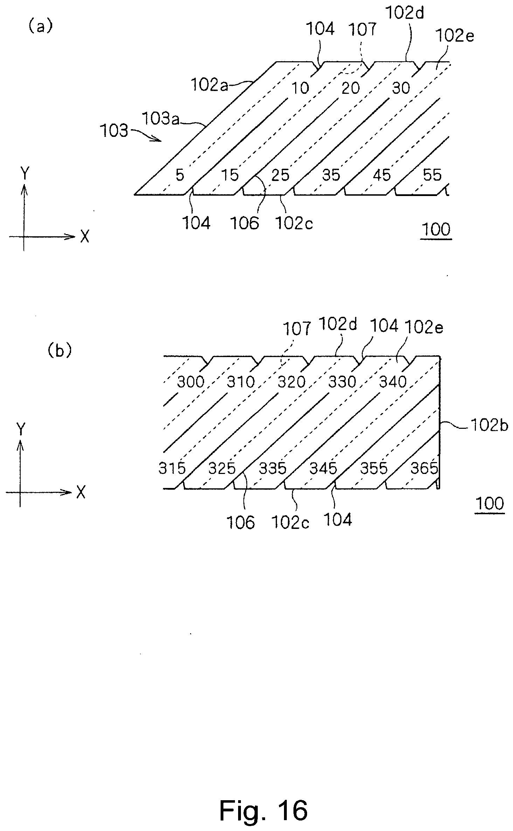

Figs. 16(a) and (b) illustrate a hair measurement tool 100 according to a tenth modification. As compared with the hair measurement tool 70 of the seventh modification of Figs. 11(b) and (c), the hair measurement tool 100 has a form that the curved parts are modified to be straight. Namely, with reference to the hair measurement tool 100, one end part 102a is formed as a mere oblique straight line part 103, and no concave is formed at a center part 103a (moreover, although the angle of the oblique straight line part 103 to side surfaces 102c, 102d is 45 degrees, the angle is not limited to this value).

Furthermore, scale lines 106 and intermediate lines 107 indicated on one surface 102e of the hair measurement tool 100 are also oblique straight lines parallel to the oblique straight line part 103 of the one end part 102a. These scale lines 106 and the intermediate scale lines 107 are indicated on the one surface 102e from the one end part 102a to another end part 102b, and discontinue at the other end part 102b, since the other end part 102b is in a straight line perpendicular to side surfaces 102c and 102d. Moreover, another surface on the reverse side of the one surface 102e of the hair measurement tool 100 is substantially the same as the one surface 102e, and notches 104 are formed in the side surfaces 102c, 102d, at the positions corresponding to the scale lines 106, likewise the cases as described above.

Since the scale lines 106 and the intermediate scale lines 107 of the hair measurement tool 100 are straight oblique lines, the user can sensuously determine the cut length without difficulties, and this is suitable for users who cannot manage the hair measurement tool 70 of the seventh modification.

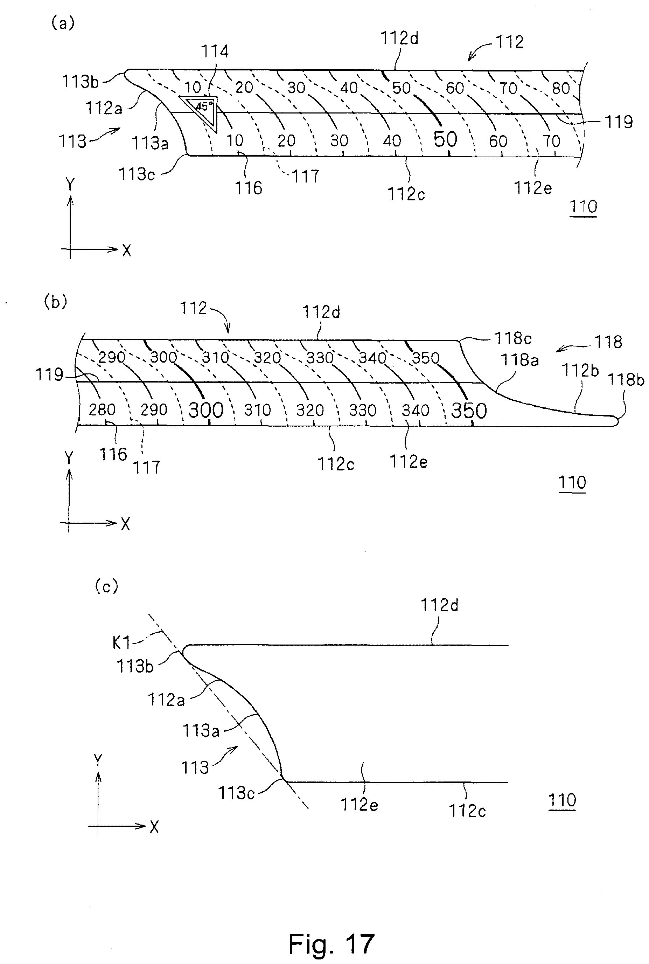

Fig. 17 through Fig. 21 illustrate a hair measurement tool 110 according to an eleventh modification, as a further modification of the hair measurement tool 70 of the seventh modification of Figs. 11(b) and (c), suitable for hair length measurement, etc., along an angular direction of a desired hair setting. Namely, as illustrated in Figs. 17(a) and (b), one end part 112a of an elongated member 112 of the hair measurement tool 110 according to the eleventh modification is aligned with an imaginary oblique line K1 at the angle of 45 degrees to the X axis as illustrated in Fig. 17(c) (when referring to the hair measurement tool 70 of Figs. 11(a) and (b) as the standard, corresponding to 135 degrees relative to the X axis), and a curved concave part 113 is formed so that an approximate center 113a is the innermost part, and in addition, at an end part 113b on the side of one side surface 112d, the curve direction changes to form a projecting curve. Therefore, the one end part 112a has a combined curved line, composed of a recessing curved line of the concave part 113, and a projecting curved line of the end part 113b of the one side surface 112d, connected at the inflection point, so that the imaginary straight oblique line K1 is tangent to the one end part 113b and another end part 113c. Moreover, as illustrated in Fig. 17(a), the hair measurement tool 110 has a mark 114 indicating that the angle relative to the one end part 112a is 45 degrees, on one surface 112e in the vicinity of the one end part 112a.

As illustrated in Fig. 17(a), scale lines 116 and intermediate scale lines 117, in the same curve as that of the one end part 112a, are indicated on the one surface 112e of the hair measurement tool 110. These scale lines 116 and the intermediate scale lines 117 are indicated with predetermined intervals from the one end part 112a to another end part 112b, likewise the case of the other embodiment and modifications as described above. Moreover, the hair measurement tool 110 indicates a center line 119 from the one end part 112a to the other end part 112b, at the center in the width direction of the one surface 112e. And furthermore, distances from the one end part 112a are indicated on each of the scale lines 116 of the hair measurement tool 110, on both the sides as seen from the center line 119.

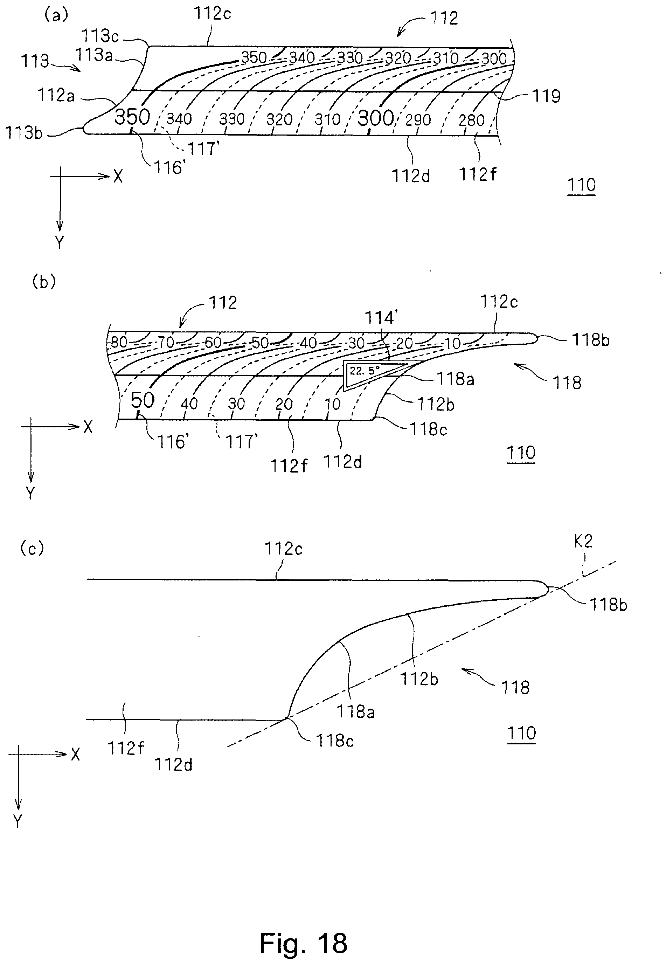

Furthermore, as illustrated in Fig. 17(b), the shape of the other end part 112b of the hair measurement tool 110 is based on an inclined angle different from that of the one end part 112a. Namely, as illustrated in Fig. 18(c), the other end part 112b of the hair measurement tool 110 is aligned with an imaginary oblique line K2 at the angle of 22.5 degrees to the X axis, and a curved concave part 118 is formed so that an approximate center 118a is the innermost part, and at an end part 118b on the side of the other side surface 112c, the curve direction changes to form a projecting curve. Since this shape of the other end part 112b on the one surface 112e is different from the curved lines of the scale lines 116 and the intermediate scale lines 117 as described above, an area without indication of the scale lines 116 or the intermediate scale lines 117 exists in the proximity of the other end part 112b on the one surface 112e. Moreover, Fig. 17(b) illustrates the vicinity of the other end part 112b as seen from the one surface 112e, while Fig. 18(c) illustrates the vicinity of the other end part 112b as seen from another surface 112f.

As described above, the other end part 112b has a combined curved line, composed of a recessing curved line of the concave part 118, and a projecting curved line of the end part 118b of the other side surface 112c, connected at the inflection point, so that the imaginary straight oblique line K2 is tangent to the one end part 118b on the side of the other side surface 112c and another end part 118c on the side of the one side surface 112d (see Fig. 18(c)).

Furthermore, as illustrated in Figs. 18(a) and (b), the other surface 112f of the hair measurement tool 110 indicates scale lines 116' and intermediate scale lines 117' in the same curve as that of the other end part 112b. The scale lines 116' and the intermediate scale lines 117' are indicated with predetermined intervals from the other end part 112b to the one end part 112a, showing distances from the other end part 112b on each of the scale lines 116', on both the sides as seen from the center line 119. Moreover, as illustrated in Fig. 18(b), the hair measurement tool 110 has a mark 114' indicating that the angle to the other end part 112b is 22.5 degrees, on the other surface 112f in the vicinity of the other end part 112b. Moreover, since the shape of curved lines of the scale lines 116' and the intermediate scale lines 117' as described above is different from the shape of the one end part 112a, as illustrated in Fig. 18(a), an area without indication of the scale lines 116' or the intermediate scale lines 117' exists in the proximity of the one end part 112a on the other surface 112f.

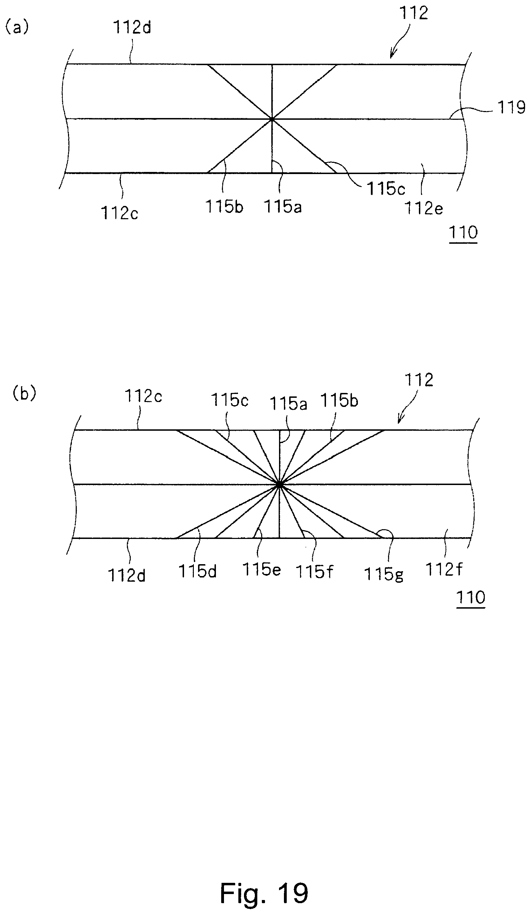

Fig. 19(a) illustrates a plurality of first through third angled lines 115a-115c, respectively indicated at the center between the one end part 112a and the other end part 112b on the one surface 112e of the hair measurement tool 110. The first angled line 115a is a straight line perpendicular to the center line 119 (parallel to the Y axis), the second angled line 115b is a straight line inclined at the angle of 45 degrees to the center line 119, and the third angled line 115c is a straight line inclined at the angle of 45 degrees to the center line 119 on the side opposite to the second angled line 115b (the third angled line 115c and the second angled line 115b are symmetry with respect to the center line 119, and the third angled line 115c is parallel to the imaginary oblique lie K1 as illustrated in Fig. 17(c)). The first through third angled lines 115a-115c are indicated at three positions in total on the one surface 112e with intervals, in particular, at the positions about 140 mm, about 240 mm and at about 340 mm, respectively from the one end part 112a, and at these three positions, the direction of picking the hair can be confirmed by the first through third angled lines 115a-115c.

Fig. 19(b) illustrates a plurality of first through seventh angled lines 115a-115g, respectively indicated at the center between the one end part 112a and the other end part 112b on the other surface 112f of the hair measurement tool 110. The first through third angled lines 115a-115c are the same as those on the one surface 112e as described above. Furthermore, the fourth angled line 115d is a straight line inclined at the angle of 22.5 degrees to the center line 119 (parallel to the imaginary oblique line K2 as illustrated in Fig. 18(c)), the fifth angled line 115e is a straight line inclined at the angle of 67.5 degrees to the center line 119, the sixth angled line 115f is a straight line inclined at the angle of 112.5 degrees to the center line 119, and the seventh angled line 115g is a straight line inclined at the angle of 157.5 degrees to the center line 119. Moreover, the fourth angled line 115d and the fifth angled line 115e, and the seventh angled line 115g and the sixth angled line 115f, are symmetry, respectively, with relation to the center line 119. The first through seventh angled lines 115a-115g are indicated at three positions in total on the other surface 112f with intervals, in particular, at the positions about 80 mm, about 180 mm and at about 330 mm, respectively from the other end part 112b, and at these three positions, the direction of picking the hair can be confirmed by the first through seventh angled lines 115a-115g.

Moreover, for the purpose of avoiding complicated drawings, Figs. 17(a) and (b) as well as Figs. 18(a) and (b) as described above omit illustration of 1-mm unit scale lines, and each of various scale lines is not illustrated in Fig. 17(c), Fig. 18(c) as well as Figs. 19(a) and (b). Furthermore, different from the hair measurement tool 70 of the seventh modification of Figs. 11(b) and (c) as described above, since the hair measurement tool 110 according to the eleventh modification does not form notches for positioning of the crest of hairdressing scissors, the elongated member 112 uses a thin material at the thickness of 1 mm for the purpose of weight saving (it is of course possible to form notches in the hair measurement tool 110 of the eleventh modification, likewise the case of the hair measurement tool 70 of the seventh modification).

Figs. 20(a) and (b) illustrate states that the hair measurement tool 110 of the eleventh modification is used for the head model H. Fig. 20(a) is a view as seen from the side of the head model H, showing the state that the one end part 112a of the hair measurement tool 110 is pressed against (placed on) the scalp Ha of the head model H. For example, when the a hair bundle h61, implanted in the vicinity of the neck at the back of the head of the head model H, is measured, for the purpose of coordinating with the natural direction of hair implantation (backward direction), the one end part 112a of the hair measurement tool 110 is pressed against the scalp Ha in a state that the one surface 112e and the side of the head model H face the same direction. At that time, since the concave part 113 is formed at the one end part 112a, the end parts 113b and 113c as illustrated in Figs. 17(a) and (c), namely the two points become in contact with the scalp Ha, thus the hair measurement tool 110 in a pressed state is maintained in a stable attitude, and the accurate measurement can be performed.

Here, as illustrated by the imaginary oblique line K1 of Fig. 17(c), since the one end part 112a is inclined at the angle of 45 degrees to the X axis, the length measurement at the angle of 45 degrees to the curved surface of the scalp Ha, into which the hair bundle h61 is implanted, can be performed easily, and when a desired hair set direction is at the angle of 45 degrees to the scalp Ha, the accurate length measurement relative to that hair set direction can be performed. In this case, since the direction at the angle of 45 degrees to the scalp position, against which the hair measurement tool 110 is pressed, is along with the direction of the center line 119, the angle of 45 degrees to the scalp can be understood easily by looking at the center line 119. Furthermore, with the first through third angled lines 115a-115c as illustrated in Fig. 19(a), with reference to the inclined direction at the angle of 45 degrees as the standard (the center line 119 serves as the standard line), the angles of 45 degrees and 135 degrees relative to the standard (the center line 119 as the standard line) can be determined by the second angled line 115b and the third angled line 115c, and the direction of picking the hair bundle in various angular directions can be measured accurately.

In addition, also in the case of measuring a hair bundle h62 implanted in the vicinity of the rear at the top of the head of the head model H, likewise the case as described above, the one end part 112a of the hair measurement tool 110 is pressed against the scalp Ha. Also in this case, the same as above, the length measurement in the direction at the angle of 45 degrees to the curved surface of the scalp Ha, into which the hair bundle h62 is implanted, can be performed securely.

Furthermore, when the lengths of hair bundles h63, h64, respectively implanted in the vicinity of the front at the top of the head model H are measured, for the purpose of coordinating with the direction of hair implantation (forward direction), the one end part 112a of the hair measurement tool 110 is pressed against the scalp Ha in a state that the other surface 112f and the side of the head model H face the same direction. Accordingly, the length measurement in the direction at the angle of 45 degrees to the curved surface of the scalp Ha, into which the hair bundles h63, h64 are implanted, can be performed securely. Moreover, further in this case, it is also possible to understand various angular directions by the angled lines 115a-115g indicated on the other surface 112f. Moreover, different from the direction of hair implantation, in the case of stand-up hair setting, in a state that the one surface 112e and the side of the head model H face the same direction, the one end part 112a is pressed against the positions of the scalp Ha into which the hair bundles h63, h64 are implanted.

Meanwhile, Fig. 20(b) is a view as seen from the front of the head model H, and when taking account of the hair set direction after haircut, the measurement is performed in a state that the one surface 112e or the other surface 112f of the hair measurement tool 110, and the front of the head model H, facing the same direction. For example, when the length of a hair bundle h71, implanted in the vicinity of the left side of the head of the head model H, is measured, in order to coordinate with the downward-oriented hair, the one end part 112a of the hair measurement tool 110 is pressed against the scalp in a state that the one surface 112e and the front of the head model H face the same direction. With the concaved part 113 of the one end part 112a, the hair measurement tool 110 maintains the stable attitude, whereby length measurement at the angle of 45 degrees relative to the curved surface of the scalp position, into which the hair bundle h71 is implanted, can be performed.

Furthermore, in regard to other hair bundles h72, h73 and h74, the one end part 112a of the hair measurement tool 110 is pressed against the scalp in a state that the one surface 112e or the other surface 112f, and the front of the head model H, facing the same direction. Accordingly, the length measurement at the angle of 45 degrees relative to the curved surfaces of the scalp positions, into which the hair bundles h72, h73 and h74 are implanted respectively, can be performed. Moreover, in the case of stand-up hair setting after haircut, the one end part 112a of the hair measurement tool 110 may be pressed against the scalp in a state that the one surface 112e or the other surface 112f, and the front of the head model H, facing the same direction, still contrary to the state as illustrated in Fig. 20(b) (for example, when the stand-up hair set of the hair bundle h72 is performed, the one end part 112a of the hair measurement tool 110 is pressed against the scalp in a state that the other surface 112f faces forward of the head).

Moreover, although Figs. 20(a) and (b) respectively illustrate the state that the one end part 112a of the hair measurement tool 110 is pressed against the scalp, where the scalp-tracing hair set after haircut is performed, it is preferable to press the other end part 112b against the scalp. In this case, as illustrated in Fig. 18(c), since the other end part 112b is inclined at the angle of 22.5 degrees, the length measurement at the angle of 22.5 degrees, to the curved surface of the scalp at the pressed position, can be performed (the direction at the angle of 22.5 degrees to the pressed position can be confirmed by the center line 119).

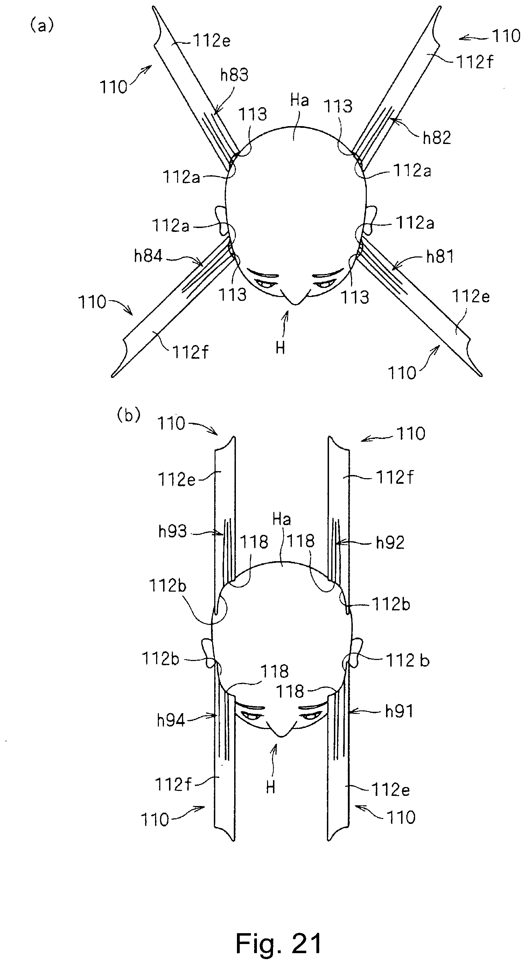

Figs. 21(a) and (b) are views as seen from the top of the head model H, showing states that the one end part 112a or the other end part 112b is pressed against the scalp Ha. Namely, Fig. 21(a) illustrates the states that the one end part 112a of the hair measurement tool 110 is pressed against the scalp Ha, wherein the one surface 112e of the hair measurement tool 110 and the top of the head model H face the same direction for hair bundles h81, h84 at cater-cornered positions, and the other surface 112f and the top of the head model H face the same direction for hair bundles h82, h83 at the other cater-cornered positions. With this pressing of the hair measurement tool 110, the length measurement at the angle of 45 degrees, namely in the natural hair direction, can be performed at the implanted position of each of the hair bundles h81 through h84 (the direction at the angle of 45 degrees is the direction of the center line 119).

Furthermore, Fig. 21(b) illustrates states that the other end part 112b of the hair measurement tool 110 is pressed against the scalp Ha, wherein the one surface 112e of the hair measurement tool 110 and the top of the head model H face the same direction for hair bundles h91, h93 at cater-cornered positions, and the other surface 112f and the top of the head model H face the same direction for hair bundles h92, h94 at the other cater-cornered positions. With this pressing of the hair measurement tool 110, the length measurement at the angle of 22.5 degrees can be performed at the implanted position of each of the hair bundles h91 through h94 (the direction at the angle of 22.5 degrees is the direction of the center line 119).

As described above, since the inclined angles of the one end part 112a and the other end part 112b of the hair measurement tool 110 of the eleventh modification are different, the length measurement at two angles (for example, 45 degrees and 22.5 degrees) relative to the scalp can be performed by appropriately changing the pressing side against the scalp (see the pressing states of Fig. 21(a) and the pressing states of Fig. 21(b)). Furthermore, when the one surface 112e and the other surface 112f are reversed appropriately, the length measurement can be performed at two more angles relative to the scalp (for example, 135 degrees against above 45 degrees, and 157.5 degrees against the above 22.5 degrees) (see Figs. 20(a) and (b)). Besides, with reference to each of the angled lines 115a-115g and the center line 119 indicated on the one surface 112e and the other surface 112f, various angles can be confirmed, whereby various picking directions of hair as the length measurement object can be confirmed.

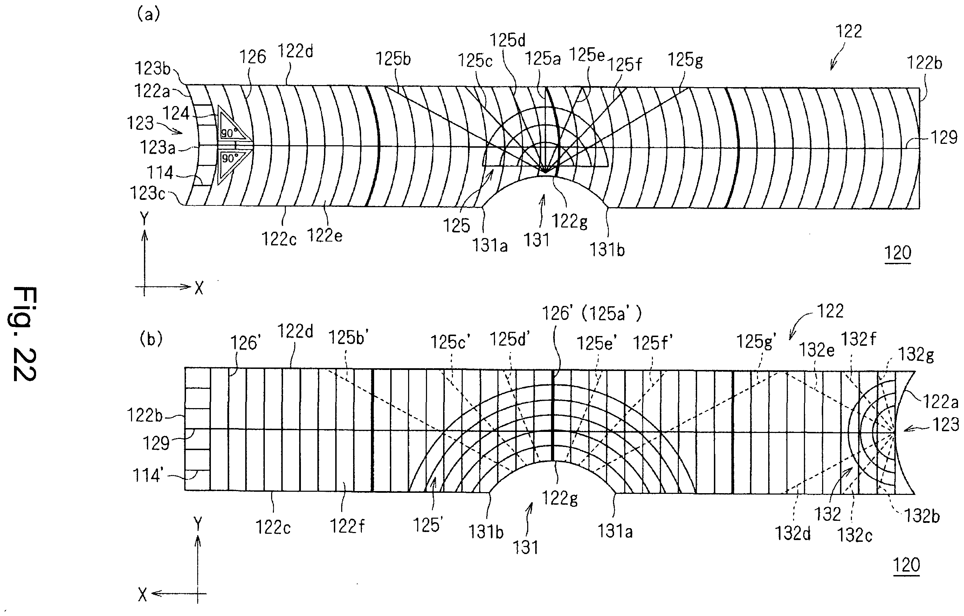

Figs. 22(a) and (b) illustrate a hair measurement tool 120 according to a twelfth modification of the present invention. The hair measurement tool 120 is a modification of the hair measurement tool 1 according to the embodiment of Fig. 1, characterized that another concave part (auxiliary concave part 131) is formed at an approximate center part 122g of one side surface 122c in the length direction of an elongated member 122 (corresponding to one part of a peripheral part of the elongated member). This auxiliary concave part 131 is an arc-shaped (fan-shaped) cutout of the approximate center part 122g, and the curvature of the arc is equivalent to that of a concave part 123 formed at one end part 122a, and it is also desirable, for example, to be equivalent to the curvature around the human neck (R=100, etc.).

Furthermore, as illustrated in Fig. 22(a), the hair measurement tool 120 indicates a protractor mark 125 above the auxiliary concave 131 on the one surface 122e, and also indicates a plurality of angled lines 125a-125g (corresponding to auxiliary angled lines) in accordance with this protractor mark 125. Each of the angled lines 125a-125g indicates the same angle as that of each of the angled lines 115a-115g illustrated in Fig. 19(b), and the intersection of the angled lines 125a-125g coincides with the center of the protractor mark 125, and also coincides with the apex of the auxiliary concave part 131. Furthermore, the hair measurement tool 120 indicates a center line 129 on the one surface 122e showing the center in the width direction, and also indicates angle marks 124 (the angle of 90 degrees relative to the length direction of the X axis) for the one end part 122a.

And furthermore, the other end part 122b of the hair measurement tool 120 is in a straight-line shape along the Y-axis direction. Furthermore, the parts other than described above on the one surface 122e of the hair measurement tool 120 are equivalent to those of the embodiment and modification as described above. In particular, likewise the case of Fig. 9(c), width scale lines 114 are indicated, and curved scale lines 126 substantially the same as those of Figs. 2(a), etc. are indicated (Figs 22(a) and (b) omit the indication of various scale lines, such as intermediate scale lines, and distances, etc.). Moreover, other than those as illustrated in Figs. 2(a) and (c), the distances from the one end part 122a may be indicated, for example, both on the side of one side surface 125c and on the side of another side surface 125d for the scale lines 126, and each of the values is indicated symmetrically by making the center line 129 as the symmetrical center. With the indication of values in this direction, the distance can be confirmed by the value on any one side, regardless of looking direction at the side surfaces 125c, 125d. Moreover, the distances regarding the intermediate scale lines may also be indicated along the center line 129.

Fig. 22(b) illustrates another surface 122f of the hair measurement tool 120. Basically, the other surface 122f also has scale lines as illustrated in Fig. 9(a), with indication of width scale lines 114' and straight scale lines 126'. Moreover, the center line 129 elongating in the X-axis direction at the center in the width direction is also indicated, and although not illustrated, distances from the other end part 122b are indicated corresponding to the scale lines 126', likewise the case of the one surface 122e.

Furthermore, the hair measurement tool 120 indicates a protractor mark 125' showing concentric arcs with predetermined intervals, for the arc-shaped curve line of the auxiliary concave part 131 formed at the approximate center part 122g in the length direction, and a plurality of angled lines 125a'-125g' (corresponding to auxiliary angled lines) in dashed lines are indicated in accordance with the center of the protractor mark 125'. The angles of the angled lines 125a'-125g' are equivalent to those of the angled lines 125a-125g indicated on the one surface 122e. Moreover, the angled line 125a' coincides with the scale line 126' at the position in the vicinity of the approximate center part 122g.

In addition, the hair measurement tool 120 also indicates an auxiliary protractor mark 132 in the vicinity of the one end part 122a on the other surface 122f, and also indicates angled lines 132b-132g, respectively elongating from the center of the auxiliary protractor mark 132. The angles of the angled lines 132b-132g are the same as those of the angled lines 125b-125g described above. Based on the angled lines 132b-132g, the hair measurement tool 120 can perform measurement related to haircut direction also at the one end part 122a. Moreover, the hair measurement tool 120 according to the twelfth modification has no notch on either side surface for the positioning of hairdressing scissors, and the thickness of the elongated member 122 made of synthetic resin, constituting the hair measurement tool 120, is about 1 mm.

Likewise the cases of Fig. 4 and Fig. 5, the hair measurement tool 120 can measure the length of the hair bundle stably with the two-point contact of both the end parts 123b, 123c of the concave part 123 by pressing the one end part 122a against each part of the scalp. Furthermore, even in the pressed state, the angle of hair bundle (the direction of picking the hair bundle) can be confirmed by the angled lines 132b-132g and the center line 129 indicated on the other surface 122f, and in particular, the direction at right angle to the pressed part of the scalp can be confirmed by the center line 129, and furthermore, the length can be measured with taking account of haircut and hair setting direction by the angled lines 132b-132g.

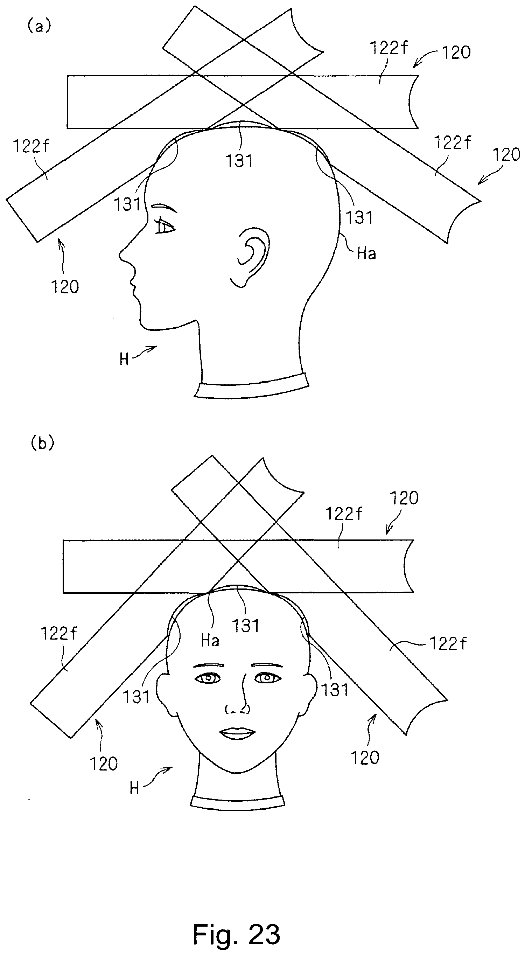

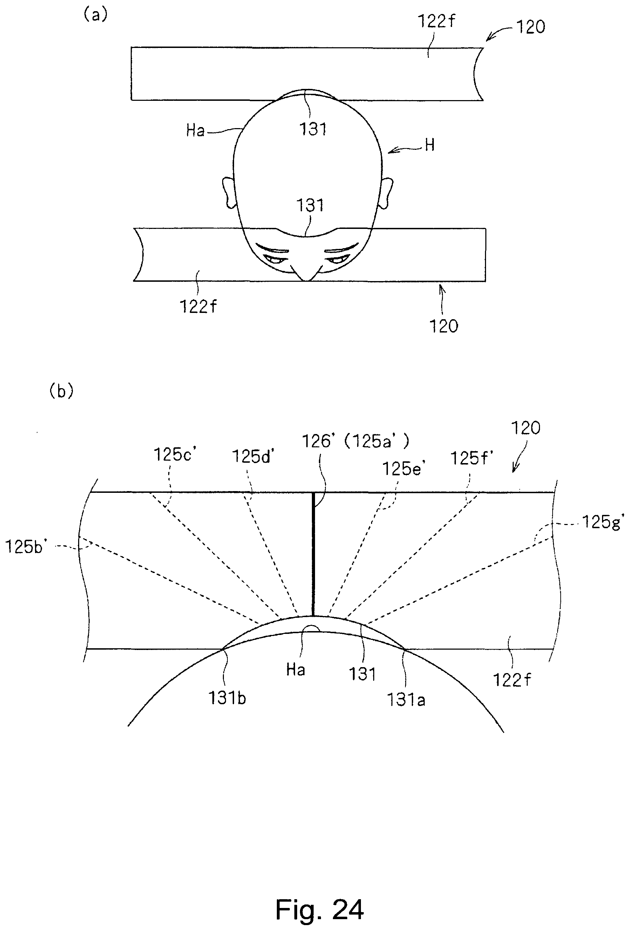

Furthermore, as illustrated in Figs. 23(a) and (b) as well as in Fig. 24(a), the hair measurement tool 120 according to the twelfth modification can measure the angular direction of hair easily and more stably. Namely, when the auxiliary concave part 131, provided at the approximate center part 122g in the length direction, is placed on the scalp Ha of the head model H, based on each of the angled lines 125a-125g indicated on the one surface 122e or each of the angled lines 125a'-125g', indicated on the other surface 122f, the measurement of haircut direction (measurement of hair angle) can be performed.

When the auxiliary concave part 131 is placed, as compared with the case that the one end part 122a is placed, since the angled lines 125b, 125c, 125f, 125g, 125b', 125c', 125f and 125g', respectively extending in the lateral direction at the angle of 22.5 degrees or 45 degrees, are indicated by longer lines, than the angled lines 132b, 132c, 132f and 132g indicated on the one end part 122a at the same angles, it is advantageous that the angle can be measured easily with the longer lines. In addition, when the auxiliary concave part 131 is placed, since the size along the direction of pressing is in the width direction of the hair measurement tool 120, the size becomes considerably shorter than the case of pressing of the one end part 122a, and therefore, the hair as the measurement object can be picked easily without being interfered by the hair measurement tool 120. Furthermore, when the auxiliary concave part 131 of the hair measurement tool 120 in the direction corresponding to the back of the head as illustrated in Fig. 24(a) is placed on the back of the neck (scruff), the direction of hair neckline (the direction of hair tips), etc., can be measured by the angled lines 125a-125g and 125a'-125g'. Furthermore, with the hair measurement tool 120 in the direction corresponding to the front of the head as illustrated in Fig. 24(a), the direction of each of hair panels (the direction of the hair tips) forming the front hair can also be measured by the angled lines 125a-125g and 125a'-125g'.

Besides, when the auxiliary concave part 131 is placed, since the hair measurement tool 120 becomes in a connecting attitude to the curved surface of the scalp, as compared with the attitude projecting in the normal line direction of the curved surface of the scalp when the one end part 122a is pressed (see Figs. 4 and 5, etc.), the user can handle the hair measurement tool 120 more easily.

And furthermore, as illustrated in Fig. 24(b), when the auxiliary concave part 131 is placed on the scalp Ha, since both end parts 131a, 131b of the auxiliary concave part 131 only become in two-point contact, the hair measurement tool 120 will not become instable in a placed state, and the measurement can be performed in a stable attitude. Accordingly, for example, when the auxiliary concave parts 131 are placed on the front, top and back of the head as seen from the side viewpoint of Fig. 23(a), or when the auxiliary concave parts 131 are placed on the left side, top and right side of the head as seen from the front viewpoint of Fig. 23(b), or when the auxiliary concave parts 131 are placed on the front and back of the head as seen from the top viewpoint of Fig. 24(a), the hair measurement tool 120 can maintain the stable attitude, whereby more accurate angle measurement can be realized. Moreover, with reference to Fig. 24(a), for the purpose of clear understanding of the direction of the head, the drawing is prepared so that the eyes and the nose can be seen through the auxiliary concave part 131 placed on the front of the head.

Moreover, the structure of the hair measurement tool 1, as well as those of the hair measurement tools 10-120 of the first through twelfth modifications may be combined arbitrarily. For example, the intermediate notches 19 for the hair measurement tool 10 of the first modification as illustrated in Fig. 8(a) may also be applied to the hair measurement tools 20-120 of the second through twelfth modifications. And besides, the notches 24 formed in the whole circumferential surfaces for the hair measurement tool 20 of the second modification as illustrated in Fig. 8(b) may also be applied to the hair measurement tool 10 of the first modification as well as to the hair measurement tools 30-120 of the third through twelfth modifications. And furthermore, the comb part 51 of the hair measurement tool 50 of the fifth modification as illustrated in Figs. 10(a) and (b) may also be applied to the hair measurement tools 70-120 of the seventh through twelfth modifications.

Furthermore, for the hair measurement tool 1 of Figs. 1, etc., the hair measurement tools 10-120 of the first through twelfth modifications, and the various combination examples described above, as a whole, it is not necessary to indicate the scale lines and the intermediate scale lines in the whole circumferential surfaces, and they may be indicated only on the necessary surfaces. In addition, when the elongated member constituting the hair measurement tool is made of transparent material, with the scale lines and the intermediate scale lines, etc., indicated only on the one surface, it is preferable that the scale lines and the intermediate scale lines, etc., can be confirmed from the transparent other surface, etc. And in addition, for the hair measurement tool 1 of Figs. 1, etc., the hair measurement tools 10-90 of the first through ninth modifications, and the various combination examples described above, as a whole, where the positioning (position restriction) of the hairdressing scissors S as illustrated in Figs, 7(b), etc., is not required and the hair (hair bundle) length measurement is the main usage, the notches (and the intermediate notches) as described above may be omitted. Furthermore, as the measurement unit of the scale lines related to the length of the various hair measurement tools 10-120 as described above, it is of course possible to apply any unit other than mm or cm, for example, inch system unit can be applied. Furthermore, the combination of different measurement system units may be also applied, for example, the scale lines and sizes may be indicated by metric system unit (mm) on the one side of the center line in the width direction, while the scale lines and the sizes may also be indicated by inch system unit on the other side of the center line in the width direction.

### INDUSTRIAL APPLICABILITY

The present invention is suitable to be applied for accurate length measurement and direction measurement of hair (hair bundle) based on the objective index, and for haircut at a desired length.

### DESCRIPTION OF REFERENCE NUMERALS

- 1: Hair Measurement Tool
- 2: Elongated Member
- 2a: One End Part
- 3: Concave Part
- 4: Notch
- 6: Scale Lines
- 31: Width Scale Lines
- 51: Comb Part
- 51a: Comb Tines
- 73: Curved Oblique Side Part
- 91: First Plate Part
- 92: Second Plate Part
- 99: One Side Part
- S: Hairdressing Scissors
- Sa: Cutting Blade
- H: Head
- Ha: Scalp
- h1, h2, h3: Hair Bundles

## Claims

1. A hair measurement tool (1), comprising:
an elongated member (2) where scale lines (6) are indicated with intervals at a predetermined length unit from one end part (2a) in a length direction thereof, and a concave part (3) is formed in the one end part (2a), and the elongated member (2) is configured, by placing the one end part (2a) on a scalp (Ha), to be used for performing hair length measurement based on the scale lines (6),
**characterized in that**
the curvature of the concave part (3) has a radius of 100 to 120 mm, so that when the one end part (2a) of the hair measurement tool (1) is pressed against the scalp (Ha), a gap from the scalp (Ha) occurs in the vicinity of the center of the concave part (3), and only end parts (3b, 3c) in a width direction of the concave part (3) contact the scalp (Ha) and both the end parts (3b, 3c) become in two-point contact with the scalp (Ha).

2. The hair measurement tool (110) of claim 1, wherein the one end part (112a) is formed to be inclined against the length direction of the elongated member (112).

3. The hair measurement tool (120) of claim 1 or 2, wherein angled lines (132b - 132g) indicating angles at the one end part (122a) are indicated on the elongated member (122), and
wherein the elongated member (122) is configured, by placing the one end part (122a) on the scalp (Ha), to be used for performing the hair direction measurement based on the angled lines (132b - 132g).

4. The hair measurement tool (120) of any one of claims 1 to 3, wherein an auxiliary concave part (131) is formed at the peripheral part (122c) along the length direction of the elongated member (122),
wherein auxiliary angled lines (125a - 125g) indicating angles at the peripheral part (122c) of the auxiliary concave part (131), are indicated on the elongated member (122), and
wherein the elongated member (122) is configured, by placing the peripheral part (122c) of the auxiliary concave part (131) on the scalp (Ha), to be used for performing the hair direction measurement based on the auxiliary angled lines (125a - 125g).

## Patentansprüche

1. Haarmessinstrument (1), umfassend:
ein längliches Element (2), wo Teilstriche (6) mit Intervallen in einer vorbestimmten Längeneinheit von einem Endteil (2a) aus in dessen Längenrichtung angegeben sind, und ein konkaver Teil (3) in dem einen Endteil (2a) ausgebildet ist, und das längliche Element (2) dafür konfiguriert ist, indem der eine Endteil (2a) auf einer Kopfhaut (Ha) platziert wird, zum Durchführen einer Haarlängenmessung basierend auf den Teilstrichen (6) verwendet zu werden,
**dadurch gekennzeichnet, dass**
die Krümmung des konkaven Teils (3) einen Radius von 100 bis 120 mm aufweist, so dass, wenn der eine Endteil (2a) des Haarmessinstruments (1) gegen die Kopfhaut (Ha) gedrückt wird, ein Spalt von der Kopfhaut (Ha) in der Umgebung der Mitte des konkaven Teils (3) auftritt und nur Endteile (3b, 3c) in einer Breitenrichtung des konkaven Teils (3) die Kopfhaut (Ha) berühren und die beiden Endteile (3b, 3c) mit der Kopfhaut (Ha) in einen Zweipunkt-Kontakt gelangen.

2. Haarmessinstrument (110) nach Anspruch 1, wobei der eine Endteil (112a) so ausgebildet ist, dass er gegen die Längenrichtung des länglichen Elements (112) geneigt ist.

3. Haarmessinstrument (120) nach Anspruch 1 oder 2, wobei gewinkelte Striche (132b - 132g), die Winkel an dem einen Endteil (122a) angeben, auf dem länglichen Element (122) angegeben sind, und
wobei das längliche Element (122) dafür konfiguriert ist, indem der eine Endteil (122a) auf der Kopfhaut (Ha) platziert wird, zum Durchführen der Haarrichtungsmessung basierend auf den gewinkelten Strichen (132b - 132g) verwendet zu werden.

4. Haarmessinstrument (120) nach einem der Ansprüche 1 bis 3, wobei ein zusätzlicher konkaver Teil (131) am peripheren Teil (122c) entlang der Längenrichtung des länglichen Elements (122) ausgebildet ist,
wobei zusätzliche gewinkelte Striche (125a - 125g), die Winkel am peripheren Teil (122c) des zusätzlichen konkaven Teils (131) angeben, auf dem länglichen Element (122) angegeben sind, und
wobei das längliche Element (122) dafür konfiguriert ist, indem der periphere Teil (122c) des zusätzlichen konkaven Teils (131) auf der Kopfhaut (Ha) platziert wird, zum Durchführen der Haarrichtungsmessung basierend auf den zusätzlichen gewinkelten Strichen (125a - 125g) verwendet zu werden.

## Revendications

1. Outil de mesure de cheveu (1) comprenant :
un organe allongé (2) sur lequel des lignes de graduation (6) sont indiquées avec des intervalles à une unité de longueur prédéterminée, depuis une partie d'extrémité (2a) dans une direction longitudinale dudit organe, et une partie concave (3) est formée dans ladite partie d'extrémité (2a), et l'organe allongé (2) est configuré en plaçant ladite partie d'extrémité (2a) sur un cuir chevelu (Ha), pour être utilisé pour réaliser une mesure de longueur des cheveux sur la base des lignes de graduation (6),
**caractérisé en ce que** la courbure de la partie concave (3) a un rayon de 100 à 120 mm, de sorte que lorsque ladite partie d'extrémité (2) de l'outil de mesure des cheveux (1) est pressé contre le cuir chevelu (Ha), un espace par rapport au cuir chevelu (H) se forme à proximité du centre de la partie concave (3), et seules les parties d'extrémité (3b, 3c) dans une direction de largeur de la partie concave (3) touchent le cuir chevelu (Ha), et les parties d'extrémité (3b, 3c) viennent toutes les deux en contact en deux points avec le cuir chevelu (Ha).

2. Outil de mesure des cheveux (110) de la revendication 1, dans lequel la partie d'extrémité (112a) est formée pour être inclinée par rapport à la direction de longueur de l'organe allongé (112).

3. Outil de mesure des cheveux (120) de la revendication 1 ou 2, dans lequel des lignes angulaires (132b-132g) indiquant des angles sur la partie d'extrémité (122a) sont indiquées sur l'organe allongé (122), et
dans lequel l'organe allongé (122) est configuré en plaçant la partie d'extrémité (122a) sur le cuir chevelu (Ha), pour être utilisé pour réaliser la mesure de direction des cheveux sur la base des lignes angulaires (132b-132g).

4. Outil de mesure des cheveux (120) de l'une quelconque des revendications 1 à 3, dans lequel une partie concave auxiliaire (131) est formée sur la partie périphérique (122c) le long de la direction de longueur de l'organe allongé (122),
dans lequel des lignes angulaires auxiliaires (125a-125g) indiquant des angles sur la partie périphérique (122c) de la partie concave auxiliaire (131) sont indiquées sur l'organe allongé (122), et
dans lequel l'organe allongé (122) est configuré en plaçant ladite partie périphérique (122c) de la partie concave auxiliaire (131) sur le cuir chevelu (Ha), pour être utilisé pour réaliser la mesure de direction des cheveux sur la base des lignes angulaires auxiliaires (125a-125g).
